# EUROPEAN PATENT SPECIFICATION

(11) **EP 2 859 108 B1**
(45) Date of publication and mention of the grant of the patent: **31.05.2017**
(21) Application number: 13728811.4
(22) Date of filing: 10.06.2013
(51) Int. Cl.: C12P 7/42, C12P 7/56, C12P 7/62, C12P 41/00

(54) **ENZYMATIC PROCESS FOR PRODUCING ALKYL (R)-LACTATE**
ENZYMATISCHES VERFAHREN ZUR HERSTELLUNG VON ALKYL-(R)-LAKTAT
PROCÉDÉ ENZYMATIQUE POUR LA PRODUCTION DU LACTATE-(R) D'ALKYLE

(30) Priority: 11.06.2012 GB 201210275; 07.01.2013 GB 201300219
(43) Date of publication of application: 15.04.2015
(73) Proprietor: Plaxica Limited, Redcar TS10 4RF (GB)
(72) Inventor: MARSHALL, Edward Leslie, London SW7 2AZ (GB); KEASEY, Alan, London SW7 2AZ (GB); SUTTON, David Mark, London SW7 2AZ (GB)
(74) Representative: Scott, Susan Margaret
(86) International application number: PCT/GB2013/051515
(87) International publication number: WO 2013/186540

(56) References cited:
- JEON ET AL: "Synthesis of alkyl (R)-lactates and alkyl (S,S)-O-lactyllactates by alcoholysis of rac-lactide using Novozym 435", TETRAHEDRON LETTERS, vol. 47, 2006, pages 6517-6520, XP025004806, cited in the application
- SHUKLOV ET AL: "Studies on the epimerization of diastereomeric lactides", TETRAHEDRON LETTERS, vol. 52, 30 December 2010 (2010-12-30), pages 1027-1030, XP028129681,
- TAKWA ET AL: "Rational redesign of Candida antarctica lipase B for the ring opening polymerization of D,D-lactide", CHEMICAL COMMUNICATIONS, vol. 47, 23 May 2011 (2011-05-23), pages 7392-7394, XP002682740,
- EMELYANENKO ET AL: "The thermodynamic properties of S-lactic acid", RUSSIAN JOURNAL OF PHYSICAL CHEMISTRY A, vol. 84, 2010, pages 1491-1497, XP002703599,
- YANG ET AL: "Improved preparation of D,L-lactide from D,L-lactic acid using microwave irradiation", POLYMER BULLETIN, vol. 61, 2008, pages 177-188, XP019625851,
- GROOT ET AL: "Life cycle assessment of the manufacture of lactide and PLA biopolymers from sugarcane in Thailand", THE INTERNATIONAL JOURNAL OF LIFE CYCLE ASSESSMENT, vol. 15, 2010, pages 970-984, XP002703670,
- SHUKLOV ET AL: "Ruthenium- and lipase-catalyzed inversion of L-lactates", TETRAHEDRON LETTERS, vol. 53, 13 September 2012 (2012-09-13), pages 6326-6328, XP002703671,

## Description

### Field of the invention

The present invention relates to the production of compounds containing the R-lactic acid building block, from compounds containing the S-lactic acid building block. In particular, the invention relates to the production of alkyl R-lactate, R-lactic acid, and other useful compounds containing the R-lactic acid building block, such as R,R-lactide and poly-R-lactic acid.

### Background of the invention

Lactic acid (2-hydroxypropanoic acid) and its cyclic dimer lactide (3,6-dimethyl-1,4-dioxan-2,5-dione) are important building blocks for the chemical and pharmaceutical industries. One example of their use is in the manufacture of polylactic acid, a polymer whose ability to be produced from a variety of renewable feedstocks and biodegradability makes it an attractive candidate to replace more conventional petrochemical polymers. Polylactic acid can be prepared via a multi-step process involving (a) dehydration of lactic acid to produce lactide (via an oligomerisation-depolymerisation process), and (b) polymerisation of the lactide under carefully controlled conditions to ensure that long polymer chains are produced in preference to shorter oligomers. Alternatively polylactic acid can be prepared directly from the condensation polymerisation of lactic acid, though obtaining high molecular weight material is generally more difficult than the ring-opening polymerisation of lactide.

Today virtually all large scale production of the lactic acid available commercially is carried out by fermentation processes, see for example Strategic Analysis of the Worldwide Market for Biorenewable Chemicals M2F2-39, Frost and Sullivan, 2009. Lactic acid is chiral and can be made in two enantiomeric forms, respectively L-lactic acid (hereinafter referred to as S-lactic acid) on the one hand and D-lactic acid (hereinafter R-lactic acid) on the other. Conventional fermentation technologies principally generate S-lactic acid with little R-lactic acid being formed. Although alternative bacterial strains, often genetically engineered, can be used to produce R-lactic acid, to date the modified bacteria and the associated processes are expensive and difficult to use reliably on a large industrial scale. This is evidenced in the comparatively high price and limited availability of R-lactic acid.

Lactide, the cyclic dimer of lactic acid, exists in three different stereoisomeric forms: R,R-lactide, S,S-lactide, and R,S-lactide. R,R-lactide and S,S-lactide are enantiomers of each other, and a mixture containing substantially equal quantities of R,R- and S,S- lactide is referred to as *rac*-lactide. R,S-lactide is also known as *meso*-lactide, and is a diastereoisomer of R,R- and S,S-lactide.

Since the most readily available source of lactic acid is S-lactic acid, the principal lactide employed commercially to date has been S,S-lactide and the polymer produced poly-L-lactic acid (PLLA) (hereinafter poly S-lactic acid). However the physical and chemical properties of poly-S-lactic acid are limited relative to conventional polymers, as are those of the corresponding poly-D-lactic acid (PDLA) (hereinafter poly R-lactic acid) which to date has limited their utility, particularly with respect to more durable and/or engineering applications.

It has been found that deficiencies can be overcome by using mixtures of poly S-lactic acid and poly R-lactic acid which are prepared by, for example, melt blending. It is believed that in these so-called 'stereocomplex' polymer mixtures close packing of the poly S-lactic acid and poly R-lactic acid chains occasioned by their differing chirality improves polymer crystallinity which leads to improvements in the properties referred to above. This permits the use of stereocomplex polylactic acid for a much wider range of consumer durable applications, making it a viable alternative to traditional commodity polymers such as polyethylene terephthalate, polypropylene and polystyrene. This approach however requires access to large quantities of poly R-lactic acid and therefore ultimately to large quantities of R-lactic acid, or to suitable derivatives thereof.

A process which permits the efficient production of such materials would be desirable. Jeon et al (Tetrahedron Letters, 47, (2006), 6517-6520) disclose the laboratory observation that *rac*-lactide can be alcoholised with various alcohols in the presence of a solvent and the supported lipase enzyme Novozym 435, to produce a mixture containing the corresponding alkyl R-lactate and alkyl S,S-lactyllactate. However, this reference does not deal with the problem of providing an efficient and economic process for producing R-lactic acid-based materials on an industrial scale. The Jeon document is also silent regarding how unwanted byproducts from the process may be utilised.

In contrast, the present inventors have identified a flexible and efficient processing solution which permits the production of R-lactic acid-based materials, which makes use of a wide variety of comparatively cheap and readily available S-lactic acid-based feedstocks, and which provides processing solutions that allow recovery and re-use of valuable lactic acid-containing byproducts.

### Summary of the invention

Accordingly, the present invention provides a process for producing alkyl R-lactate comprising: producing an intermediate comprising R,R- and S,S-lactide from an initial compound comprising substructure (I) 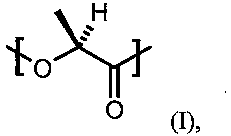 wherein the initial compound is subjected to stereoisomerisation conditions, and wherein the initial compound is contacted with a basic or acidic stereoisomerisation catalyst, resulting in a change from a first stereoisomeric distribution to a second different stereoisomeric distribution; and reacting at least a portion of the intermediate with an alkyl alcohol to produce a product comprising alkyl R-lactate, wherein alkyl R-lactate is produced in the presence of an enzyme.

The invention also provides a process for producing alkyl S,S-lactyllactate comprising: producing an intermediate comprising R,R- and S,S-lactide from an initial compound comprising substructure (IP) 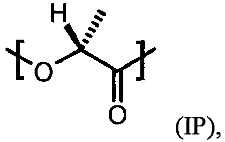 wherein the initial compound is subjected to stereoisomerisation conditions, and wherein the initial compound is contacted with a basic or acidic stereoisomerisation catalyst, resulting in a change from a first stereoisomeric distribution to a second different stereoisomeric distribution; and
reacting at least a portion of the intermediate with an alkyl alcohol to produce alkyl S,S-lactyllactate and alkyl R,R-lactyllactate, wherein the alkyl R,R-lactyllactate is reacted with alkyl alcohol in the presence of an enzyme to produce alkyl R-lactate.

### Brief description of the drawings

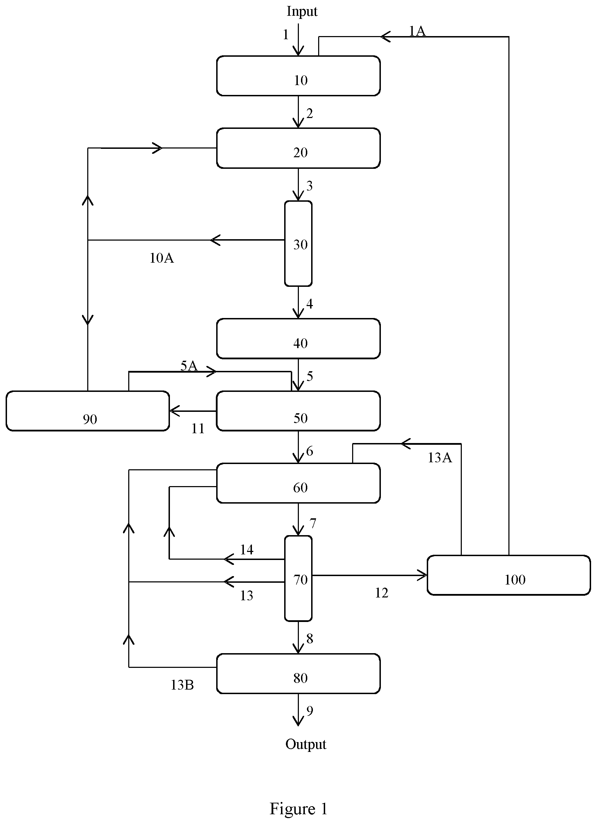

Figures 1 to 3 show embodiments of the process of the invention.

### Detailed description

### Definitions:

**Lactic acid:** A compound having the formula

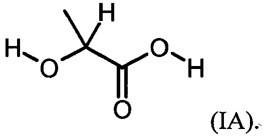

The term lactic acid encompasses S-lactic acid, R-lactic acid, and mixtures thereof, including racemic lactic acid (a mixture containing substantially equal proportions of S-lactic acid and R-lactic acid, i.e. about 50:50), and scalemic lactic acid mixtures (mixtures containing S-lactic and R-lactic acid in non-equal proportions).

**R-lactic acid:** A compound having the formula

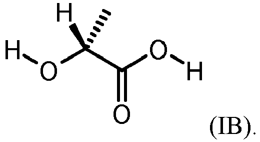

**S-lactic acid:** A compound having the formula

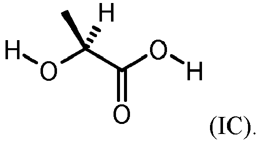

**Lactide:** A compound having the formula

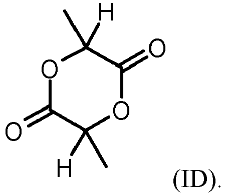

The term lactide encompasses R,R-lactide, S,S-lactide, R,S-lactide, and mixtures thereof, such as racemic lactide (a mixture containing substantially equal proportions of S,S-lactide and R,R-lactide, i.e. about 50:50).

**R,R-lactide:** A compound having the formula

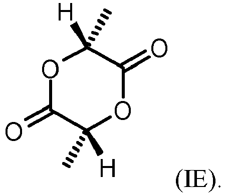

**S,S-lactide: A** compound having the formula

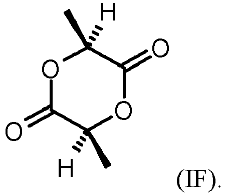

**R,S-lactide: A** compound having the formula 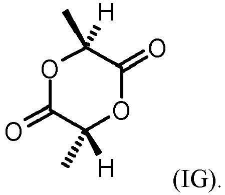 R,S-lactide is also known as *meso*-lactide.

**Racemic-lactide:** A mixture containing substantially equal proportions of S,S-lactide and R,R-lactide (i.e. about 50:50), also referred to as *rac*-lactide.

**Oligomeric S-lactic acid:** Oligomer of lactic acid (sometimes referred to as a pre-polymer) containing S-lactic acid monomer units, i.e. a compound containing the substructure 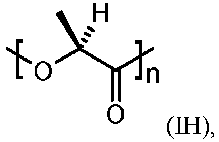 in which n typically represents an integer of up to 50. In some embodiments, oligomeric S-lactic acid may terminate in a carboxylic acid group. In other embodiments, for example where oligomeric S-lactic acid is produced by oligomerisation of alkyl S-lactate, oligomeric S-lactic acid may terminate in an alkyl ester group.

**Oligomeric R-lactic acid:** Oligomer of lactic acid (sometimes referred to as a pre-polymer) containing R-lactic acid monomer units, i.e. a compound containing the substructure 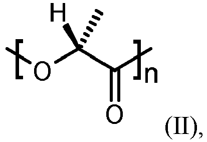 in which is n typically represents an integer of up to 50. In some embodiments, oligomeric R-lactic acid may terminate in a carboxylic acid group. In other embodiments, for example where oligomeric R-lactic acid is produced by oligomerisation of alkyl R-lactate, oligomeric R-lactic acid may terminate in an alkyl ester group.

**Oligomeric lactic acid:** Oligomer of lactic acid (sometimes referred to as a pre-polymer) comprising both R- and S-lactic acid monomer units. Oligomeric lactic acid typically contains up to 50 lactic acid monomer units. In some embodiments, oligomeric lactic acid may terminate in a carboxylic acid group. In other embodiments, for example where oligomeric lactic acid is produced by oligomerisation of alkyl lactate, oligomeric lactic acid may terminate in an alkyl ester group.

**Alkyl lactyllactate:** A compound having the formula 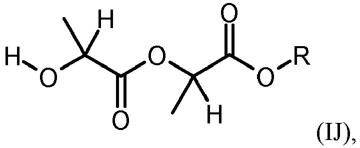 in which R represents an alkyl group. The term alkyl lactyllactate encompasses alkyl R,R-lactyllactate, alkyl S,S-lactyllactate, alkyl S,R-lactyllactate, alkyl R,S-lactyllactate, and mixtures thereof.

**Alkyl S,S-lactyllactate:** A compound having the formula 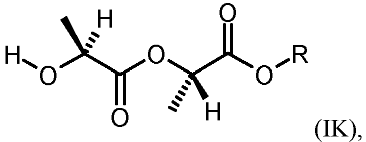 in which R represents an alkyl group.

**Alkyl R,R-lactyllactate:** A compound having the formula 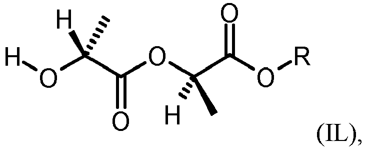 in which R represents an alkyl group.

**Alkyl lactate: A** compound having the formula 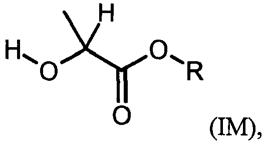 in which R represents an alkyl group. The term alkyl lactate encompasses alkyl S-lactate, alkyl R-lactate, and mixtures thereof, including racemic alkyl lactate (a mixture containing substantially equal quantities of alkyl S-lactate and alkyl R-lactate), and scalemic alkyl lactate mixtures (mixtures containing alkyl S-lactate and alkyl R-lactate in non-equal proportions).

**Alkyl S-lactate: A** compound having the formula 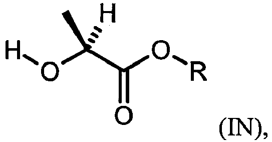 in which R represents an alkyl group.

**Alkyl R-lactate:** A compound having the formula 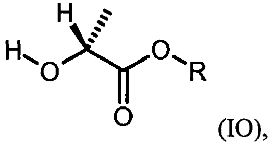 in which R represents an alkyl group.

The process of claim 1 provides a flexible and economic process which provides alkyl R-lactate in high enantiomeric purity, using S-lactic acid based materials as the feedstock. Typically the enantiomeric excess of the alkyl R-lactate produced by the process is at least 80%, preferably at least 90%, more preferably at least 95%, still more preferably at least 98%, most preferably at least 99%.

A particular advantage is the flexibility of the process, in that a variety of lactic acid-based building blocks may be used as feedstocks, including waste materials, byproducts or polylactic acid from other lactic acid-based processes. The initial compound comprises substructure (I), i.e. the initial compound incorporates a building block based on S-lactic acid. For example, the initial compound may be oligomeric S-lactic acid (e.g. oligomeric S-lactic acid terminating in a carboxylic acid end group, or terminating in an alkyl ester group), S-lactic acid or a salt thereof (e.g. ammonium lactate), alkyl S-lactate (e.g. n-butyl S-lactate or methyl S-lactate), alkyl S,S-lactyllactate and/or S,S-lactide. Any of those materials mixed with the corresponding R-material may also be used. Thus for example, waste lactic acid feedstocks containing S-lactic acid as a major constituent together with R-lactic acid as a minor constituent may be used. Similarly, oligomeric lactic acid comprising a majority of S-lactic acid monomer units and a minority of R-lactic acid monomer units may be utilised. Thus, the initial compound may, in addition to comprising substructure (I), comprise substructure (IP), 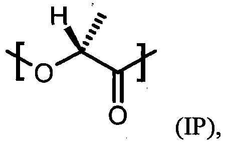 i.e. the initial compound may also comprise a building block based on R-lactic acid. Polylactic acid, for example poly S-lactic acid (e.g. waste poly S-lactic acid of insufficient chiral purity to be useful as a commercial product, or recycled polylactic acid), may be used. Other possible feedstocks include polylactic acid plant waste streams (containing e.g. lactide monomer, oligomeric lactic acid and/or meso-lactide). A single initial compound or a combination of initial compounds may be used. The process involves subjecting an initial compound comprising substructure (I) to stereoisomerisation conditions. This has the effect of randomising the stereochemical configuration at chiral centres containing an acidic proton within the compound comprising substructure (I). Stereoisomerisation may also be termed 'racemisation' but is not limited to the production of an optically inactive racemate - in other words, stereoisomerisation can produce a mixture of optical isomers of non-equal amounts, i.e. a partial racemisation. For example, when the initial compound is alkyl S-lactate, subjecting alkyl S-lactate to stereoisomerisation conditions leads to the production of a mixture of alkyl R-lactate and alkyl S-lactate. When the initial compound is S-lactic acid, subjecting the S-lactic acid to stereoisomerisation conditions leads to the production of a mixture of R-lactic acid and S-lactic acid. When the initial compound, is oligomeric S-lactic acid, subjecting the oligomeric S-lactic acid to stereoisomerisation conditions leads to the production of oligomeric lactic acid (i.e. oligomer of lactic acid comprising both R- and S-lactic acid monomer units). When the initial compound is alkyl S,S-lactyllactate, subjecting the alkyl S,S-lactyllactate to stereoisomerisation conditions leads to the production of alkyl lactyllactate (i.e. a mixture comprising alkyl R,R-lactyllactate, alkyl S,S-lactyllactate, alkyl R,S-lactyllactate and alkyl S,R-lactyllactate).

Subjecting the initial compound comprising substructure (I) to stereoisomerisation conditions results in a change from a first stereoisomeric distribution to a second different stereoisomeric distribution.

The initial compound is contacted with a basic stereoisomerisation catalyst, which is preferred, or an acidic stereoisomerisation catalyst. A preferred stereoisomerisation catalyst is an amine catalyst, preferably a tertiary amine catalyst, more preferably a trialkylamine wherein each alkyl group independently comprises 2 to 12 carbon atoms, most preferably triethylamine. Other examples of preferred amine catalysts include ammonia, trimethylamine, tri(n-propyl)amine, tri(n-butyl)amine, tri(n-pentyl)amine, tri(n-hexyl)amine, tri(n-octyl)amine, tri(n-decyl)amine, Alamine 336, pyridine, lutidine, dimethylamine, diethylamine, di(n-propyl)amine, di(iso-propyl)amine, di(n-butyl)amine, di(tert-butylamine), diphenylamine, methylamine, ethylamine, n-propylamine, iso-propylamine, n-butylamine, sec-butylamine, iso-butylamine, tert-butylamine, n-pentylamine, n-hexylamine, 2-ethylhexylamine, n-octylamine, n-decylamine, phenylamine (aniline), benzylamine. Such amine catalysts are particularly suitable for use where the initial compound is oligomeric lactic acid (e.g. oligomeric S-lactic acid, for example oligomeric S-lactic acid terminating in an acid end group, or terminating in an alkyl ester end group), alkyl lactate (e.g. alkyl S-lactate), alkyl lactyllactate (e.g. alkyl S,S-lactyllactate) and/or lactide (e.g. R,S-lactide). The amine-catalysed stereoisomerisation reaction can be carried out at any temperature suitable to effect randomisation of chiral centres within the compound having substructure (I), for example it may be carried out at a temperature of from 80 to 220°C, more preferably from 100 to 200°C, most preferably from 120 to 180°C.

Another suitable stereoisomerisation catalyst is a metal alkoxide catalyst, preferably a C₁ to C₈ metal alkoxide, more preferably sodium n-butoxide. Metal alkoxides are particularly suitable for use with initial compounds such as alkyl lactate. In that case, the metal alkoxide is preferably selected so that the alcohol from which the alkoxide is derived corresponds to the alcohol from which the lactate ester is derived, so that any transesterification that may take place does not result in the formation of mixtures of alkyl lactates having different ester groups. In some circumstances, such metal alkoxides may also be preferred for use with initial compounds where simultaneous alcoholysis and randomisation of chiral centres is desired, for example with alkyl lactyllactate (e.g. alkyl S,S-lactyllactate) as the initial compound. Alternatively, where it is desired to reduce or avoid transesterification and/or alcoholysis, a sterically hindered metal alkoxide may be employed, such as potassium or sodium t-butoxide. Where the stereoisomerisation catalyst is a metal alkoxide, the stereoisomerisation may be carried out at any temperature suitable to effect randomisation of chiral centres within the compound having substructure (I), for example it may be carried out at a temperature of from 25 to 150°C, more preferably from 50 to 100°C. Further examples of suitable catalysts include metal salts such as metal stearates, preferably calcium stearate. Where the initial compound is lactic acid, the catalyst may be a metal hydroxide, such as sodium hydroxide.

In some embodiments, where the initial compound is lactic acid, suitable stereoisomerisation conditions may involve exposing the initial compound, to high temperature (e.g. from 250 to 325°C) and high pressure conditions (e.g 150 bar (15,000,000 Pa)), preferably in the presence of a metal hydroxide catalyst, such as sodium hydroxide.

Where the initial compound is other than lactide the initial compound is converted into the intermediate via a process involving (i) subjecting the initial compound (e.g. oligomeric S-lactic acid, S-lactic acid or a salt thereof, alkyl S,S-lactyllactate and/or alkyl S-lactate) to stereoisomerisation conditions; and (ii) converting at least a portion of the product of step (i) into the intermediate comprising R,R- and S,S-lactide. Steps (i) and (ii) may be carried out either in a single vessel, or separately.

In a preferred embodiment, the initial compound comprises oligomeric-S-lactic acid, and step (i) comprises contacting the oligomeric S-lactic acid with a tertiary amine stereoisomerisation catalyst at a temperature of from 100 to 200°C to produce oligomeric lactic acid. The oligomeric S-lactic acid may for example be produced from S-lactic acid (e.g. by heating S-lactic acid under dehydrating conditions), or it may be produced from alkyl S,S-lactyllactate and/or alkyl S-lactate (e.g. by heating and distilling off alcohol). Where the oligomeric S-lactic acid is produced from S-lactic acid, the S-lactic acid may, for example, be produced from alkyl S,S-lactyllactate and/or alkyl S-lactate (e.g. by hydrolysis). In the preferred embodiment wherein the initial compound comprises oligomeric S-lactic acid, preferably step (ii) comprises converting at least a portion of the oligomeric lactic acid into R,R- and S,S-lactide by heating in the presence of a Lewis acid catalyst comprising an oxide, alkoxide or carboxylate salt of a metal. More preferably the Lewis acid catalyst is selected from the group consisting of tin(II)bis(octanoate), Sb₂O₃, ZnO, tin(II)bis(2-ethylhexanoate), Al(OiPr)₃, Al(lactate)₃, Zn(lactate)₂, Sn(lactate)₂, Ti(OiPr)₄, Ti(OnBu)₄, MgO, CaO, Mg(lactate)₂, Ca(lactate)₂, and Y(OCH₂CH₂NMe₂)₃; still more preferably the Lewis acid catalyst is tin(II)bis(octanoate), ZnO or Al(OiPr)₃; most preferably tin(II)bis(octanoate). The reaction conditions (e.g. temperature and pressure) may be selected so as remove lactide from the reaction mixture by distillation as it is formed, for example the temperature may be from 120 to 250°C, more preferably from 160 to 240°C, still more preferably from 180 to 220°C, preferably at a pressure of from 5000 Pa to 1000 Pa.

Preferably, the oligomeric S-lactic acid is produced from S-lactic acid by oligomerisation in an evaporator unit, such as a falling film evaporator or a wiped film evaporator. The continual evaporation process facilitates the removal of water from the condensation of S-lactic acid and/or S-oligomeric lactic acid fragments. Advantageously, stereoisomerisation (i.e. step (i)) and/or subsequent removal of tertiary amine catalyst may also be performed in the same vessel. Conversion of oligomer to lactide (i.e. step (ii)) may also be carried out in an evaporator unit, such that steps (i) and (ii) may be operated in one or more evaporator units.

In an alternative but still preferred embodiment, the initial compound comprises S-lactic acid, and step (i) comprises esterifying the S-lactic acid under stereoisomerisation conditions to produce alkyl R-lactate and alkyl S-lactate. In another preferred embodiment, the initial compound comprises alkyl S-lactate, and step (i) comprises contacting the alkyl S-lactate with a tertiary amine or, more preferably, with a metal alkoxide stereoisomerisation catalyst to produce alkyl R-lactate and alkyl S-lactate, for example at a temperature of from 25 to 150°C, more preferably from 80 to 150°C. In those embodiments, preferably step (ii) comprises converting the alkyl R-lactate and alkyl S-lactate into oligomeric lactic acid (e.g. by heating and removing alcohol by distillation to produce oligomeric lactic acid terminating in an alkyl ester end group), and converting at least a portion of the oligomeric lactic acid into R,R- and S,S-lactide by heating in the presence of a Lewis acid catalyst comprising an oxide, alkoxide or carboxylate salt of a metal. Preferred Lewis acid catalysts and conditions are the same as those indicated above for processes based on oligomeric S-lactic acid as the initial compound.

In another preferred embodiment, the initial compound comprises alkyl S,S-lactyllactate, and step (i) comprises contacting the alkyl S,S-lactyllactate with a tertiary amine stereoisomerisation catalyst at a temperature of from 80 to 220°C, more preferably from 100 to 200°C, most preferably from 120 to 180°C to produce alkyl lactyllactate. In that embodiment, step (ii) preferably comprises converting the alkyl lactyllactate into oligomeric lactic acid, and converting at least a portion of the oligomeric lactic acid into R,R- and S,S-lactide by heating in the presence of a Lewis acid catalyst comprising an oxide, alkoxide or carboxylate salt of a metal. Preferred Lewis acid catalysts and conditions are the same as those indicated above for processes based on oligomeric S-lactic acid as the initial compound.

Subjecting the initial compound to stereoisomerisation conditions typically leads to the production of some R,S-lactide. R,R- and S,S-lactide may be separated from R,S-lactide prior to reaction with alkyl alcohol and conversion into R-alkyl lactate, for example by crystallisation (e.g. melt crystallisation, fractional crystallisation) and/or by distillation and/or by solvent extraction/solvent washing, taking advantage of differences in solubility, boiling point and/or melting point between lactides. Suitable crystallisation solvents include ethyl acetate and toluene. Purification (e.g. crystallisation and/or distillation may also result in separation of the R,R- and S,S-lactide from other materials which may be present in the mixture, such as tertiary amine. The reaction of R,R- and S,S-lactide with alkyl alcohol may be carried out using mixtures containing small quantities of R,S-lactide, but preferably the intermediate that is reacted with alkyl alcohol is free or substantially free from R,S-lactide.

The alkyl alcohol is preferably a C₂ to C₈ alkyl alcohol, more preferably, a C₃ to C₈ alkyl alcohol, yet more preferably a C₃ to C₄ alkyl alcohol. The alcohol is preferably a linear alcohol. Ethanol, n-propanol, i-propanol, and n-butanol are preferred alkyl alcohols, with n-propanol and n-butanol being particularly preferred. In one preferred embodiment the alkyl alcohol is n-butanol. In one preferred embodiment the alkyl alcohol is n-propanol. In one preferred embodiment the alkyl alcohol is i-propanol. Alkyl alcohol may be used as solvent, in which case it is preferred that the alkyl alcohol is selected so that the R,R- and S,S-lactide are completely or partly soluble in the alkyl alcohol. Thus, in one embodiment, the intermediate is reacted with alkyl alcohol in the substantial absence of solvent other than alkyl alcohol (i.e. in that case the alcohol, the R,R- and S,S-lactide and/or the enzyme may contain some residual solvent, such as water). In other embodiments, other solvent/co-solvent may be employed, for example a solvent/co-solvent which is miscible with the alkyl alcohol. If the R,R- and S, S- lactide is insoluble or has only poor solubility/miscibility with the alkyl alcohol, it may be preferred to employ a solvent/co-solvent which is miscible with the alkyl alcohol and in which the R,R- and S,S-lactide is at least partially soluble, particularly where the process as carried out as a continuous process (e.g. a flow reactor is used). Typical preferred examples of solvent/co-solvent include unreactive oxygen-containing solvents for example dialkyl ethers (e.g. diethyl ether, dipropyl ether or MTBE) tetrahydrofuran, 1,4-dioxane, glycol ethers, polyalkylene glycol ethers and the like. Ketone solvents/co-solvents are particularly preferred. Preferred ketone solvents include methyl ethyl ketone, methyl isobutyl ketone and, in particular, acetone. A further example of a co-solvent is alkyl lactyllactate (e.g. n-butyl lactyllactate). The alkyl alcohol or the alkyl alcohol/co-solvent mixture may contain some water. In some preferred embodiments, molecular sieves are used in the process. The process may be conducted using excess alkyl alcohol together with additional solvent/co-solvent. It will be understood that the process may also be carried out using stoicheometric or even sub-stoicheometric quantities of aliphatic alcohol, and the "other" solvent may be the principal or only solvent. Typically the amount of alkyl alcohol used is such that the molar ratio of alkyl alcohol to the combined molar quantity of R,R- and S,S-lactide is in the range 1:1 to 10:1, preferably 2:1 to 5:1, more preferably 2:1 to 3:1.

The enzyme suitably comprises an esterase which is able to stereoselectively catalyse the reaction of alkyl R,R-lactyllactate with alkyl alcohol to produce alkyl R-lactate. Preferably, the esterase is a lipase. More preferably the enzyme (e.g. the esterase, lipase) is one which is either chemically or physically immobilised on a porous support for example a polymer resin bead or a silica, alumina or aluminosilicate bead, or is physically aggregated by inter-enzyme cross-linking. One particularly preferred example is *Candida antarctica* lipase B, a serine hydrolase with known enantiomeric selectivity towards the hydrolysis of esters. In this aspect of the invention, the *Candida antarctica* lipase B is most preferably chemically or physically bound to micro or nano beads made of a polymer resin for example a functionalised styrene/divinylbenzene copolymer or a polyacrylate resin, as is the case for example in the commercially available material Novozym 435. Other preferred enzymes include IMMCALB-T2-150, an immobilised *Candida antarctica* lipase B covalently attached to dry acrylic beads, manufactured by Chiralvision; IMMCALBY-T2-150, a generic *Candida antarctica* lipase B covalently attached to dry acrylic beads manufactured by Chiralvision; IMMCALB-T1-350, a *Candida antarctica* lipase B absorbed on dry polypropylene beads, manufactured by Chiralvision; and cross-linked aggregate of *Candida antarctica* lipase B, manufactured by CLEA. The enzyme may also be a recombinant *Candida antarctica* lipase B from *Aspergillus oryzae,* supplied by Sigma Aldrich (non-immobilised). Preferably the enzyme (e.g. lipase, *Candida antarctica* lipase B) is recovered and recycled to the process.

The conversion of the intermediate comprising R,R- and S,S-lactide to the product comprising alkyl R-lactate is suitably carried out at a temperature in the range of from 15 to 140°C in order to ensure that reaction rates are significant on the one hand and that the enzyme does not deteriorate with long term use on the other. Preferably the temperature employed is in the range 25 to 80°C, more preferably 30 to 70°C. In some preferred embodiments the temperature is in the range of from 40 to 75 °C.

The alkyl R-lactate produced in the process may be separated from the alkyl S,S-lactyllactate, for example by distillation, e.g. at a temperature of from 50°C to 120°C and at a pressure of from 100 Pa to 10,000 Pa. At least the lower boiling alkyl R-lactate fraction is removed overhead for further use or treatment thereby indirectly effecting separation of the two lactic acid enantiomers. Thus, either or both of alkyl R-lactate and alkyl S,S-lactyllactate may be recovered from the process of the invention. In a preferred embodiment, the alkyl R-lactate is removed overhead by distillation, and the distillation residue comprises alkyl S,S-lactyllactate, which may be removed via a side stream. In an alternative embodiment, both components are removed overhead by distillation (e.g. they are collected as separate product streams, for example at different temperatures and/or pressures). The distillation column used may optionally contain packing in order to achieve improved separation efficiencies, for example Raschig rings or structured packing.

Preferably, if the conversion of intermediate to product is operated in a batch-type reactor, the enzyme is separated (e.g. by filtration) from the reaction product before distillation, facilitating the recovery and recycle of the enzyme. For example, if a supported enzyme is used the reaction can be carried out batchwise in a single stirred or highly back-mixed tank after which the supported enzyme is separated, e.g. by filtration or the use of hydrocyclones, and the purified liquid fed to the kettle of a distillation column. In such a case the residence times of the reactants and the enzyme in the stirred tank will typically be in the range of up to 24 hours, preferably up to 10 hours, more preferably of from 1 to 8 hours, and the amount of supported enzyme used will typically be in the range up to 10%, preferably up to 5%, by weight of the intermediate comprising R,R- and S,S-lactide.

In an alternative step, the enzyme may be immobilised (e.g. Novozym-435) and stored in a packed bed column. A mixture of intermediate (e.g. R,R-lactide and S,S-lactide), alkyl alcohol (e.g. n-butanol) and solvent/co-solvent (e.g. acetone) may be passed along the column (i.e. the mixture may be brought into contact with the enzyme, e.g. Novozym-435, by passing the mixture through a packed bed of enzyme) with a residency time selected so as to ensure high conversion. This arrangement permits continuous generation of product by flow operations and separation of product and enzyme without the need for filtration of the enzyme. In a particularly preferred arrangement, the packed bed is vertical, and the mixture is fed into the top of the column. Ketone solvents/co-solvents are particularly preferred for use with such processes. Preferably, the step of reacting intermediate with alkyl alcohol is carried out continuously in a tower reactor by for example trickling the liquid reactants down through a fixed or fluidised bed of supported enzyme contained therein. A product mixture comprising alkyl R-lactate, alkyl S,S-lactyllactate and optionally unreacted lactide, unreacted alcohol, solvent/co-solvent and unreacted R,R-lactyllactate (if present) can then be recovered from the bottom of the tower. In this arrangement, the contact time of the reactants with the bed is typically in the range of up to 24 hours. Preferably residency times (contact time of the reactants with the bed) are in the range of from 10 minutes to 4 hours, more preferably from 10 minutes to 2 hours.
Arrangements of this type permit continuous or semi-continuous generation of product by flow operations.

Where the step of reacting intermediate with alkyl alcohol is operated in a batch-type reactor, the mixture containing alkyl R-lactate and alkyl S,S-lactyllactate may be separated from the enzyme by, for example, filtration of the enzyme, or by decanting or siphoning off the mixture, e.g. prior to distillation. Preferably, in the case of a batch-type process, the enzyme is re-used at least once, more preferably at least twice, still more preferably at least 5 times, yet more preferably at least 10 times, most preferably at least 20 times.
In the case of a process where R,R-lactide, S,S-lactide and alkyl alcohol are passed through a packed bed of enzyme (i.e. a continuous or semi-continuous flow process), product and enzyme are continually being separated from one another and the enzyme is continually being recycled. Accordingly, in one preferred embodiment, the process of the invention comprises: producing an intermediate comprising R,R- and S,S-lactide from an initial compound comprising substructure (I) 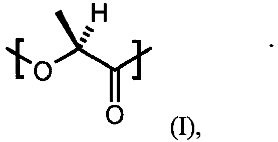 wherein the initial compound is subjected to stereoisomerisation conditions, and wherein the initial compound is contacted with a basic or acidic stereoisomerisation catalyst, resulting in a change from a first stereoisomeric distribution to a second different stereoisomeric distribution; and
reacting at least a portion of the intermediate comprising R,R-and S,S-lactide with an alkyl alcohol (e.g. n-butanol) to produce a product comprising alkyl R-lactate (and alkyl S,S-lactyllactate), by passing a solution containing R,R- and S,S-lactide, alkyl alcohol (e.g. n-butanol) and optionally solvent/co-solvent (e.g. ketone solvent/co-solvent such as acetone) through a packed bed of immobilised enzyme (e.g. Novozym 435).

A particular advantage of the process is that it allows for the possibility of recovering and recycling reagents and catalysts used in the process, and also for recycling of lactic acid-derived byproducts produced by the process, in particular R,S-lactide and alkyl S,S-lactyllactate. For example, R,S-lactide may be separated from R,R- and S,S-lactide, e.g. by crystallisation and/or by distillation and/or by solvent extraction/solvent washing as discussed above. The R,S-lactide may then be recycled, for example by contacting the R,S-lactide with a tertiary amine stereoisomerisation catalyst so as to produce further R,R- and S,S-lactide (together with R,S-lactide), which can be combined with other intermediate streams, and allows the production of additional alkyl R-lactate. Alternatively, the R,S-lactide may be recycled by hydrolysis to racemic lactic acid, which can be returned to the process by combining it with other lactic acid feedstock.

Alkyl S,S-lactyllactate may be recovered and optionally recycled to the process. As mentioned above, alkyl S,S-lactyllactate may be separated from alkyl R-lactate by distillation. In the case where the initial compound is oligomeric S-lactic acid, which oligomeric S-lactic acid is produced from an S-lactic acid feedstock (e.g. by heating S-lactic acid under dehydrating conditions), the alkyl S,S-lactyllactate may be hydrolysed to produce further S-lactic acid, which may then be returned to the process by combining it with the other S-lactic acid feedstock. Hydrolysis of the alkyl S,S-lactyllactate may also lead to production of S,S-lactyllactic acid, which may be returned to the process. Alternatively, the alkyl S,S-lactyllactate may be converted directly to oligomeric S-lactic acid (e.g. by heating and distilling off alcohol) which may be returned to the process by combining it with other oligomeric S-lactic acid feedstocks. A further option involves subjecting the alkyl S,S-lactyllactate to stereoisomerisation conditions (e.g. contacting the alkyl S,S-lactyllactate with a tertiary amine stereoisomerisation catalyst at a temperature of from 80 to 220°C, more preferably from 100 to 200°C, most preferably from 120 to 180°C) to produce alkyl lactyllactate, converting the alkyl lactyllactate into oligomeric lactic acid (i.e. oligomeric lactic acid terminating in an alkyl ester group), and combining the oligomeric lactic acid with oligomeric lactic acid produced by other routes (for example oligomeric lactic acid obtained by subjecting oligomeric S-lactic acid to stereoisomerisation conditions).

Where the stereoisomerisation catalyst is a tertiary amine, the tertiary amine may also be recycled to the process following separation from other materials (e.g. by distillation). The tertiary amine may be recovered via an optional purification step described above which may be carried out to purify the intermediate comprising R,R- and S,S-lactide, for example it may be recovered during crystallisation, distillation and/or solvent washing/extraction steps. The tertiary amine may alternatively be recovered by a purification (e.g. distillation) step carried out at an earlier stage in the process. For example, in one preferred embodiment, a tertiary amine distillation step may be carried out after step (i) (in which the initial compound is subjected to stereoisomerisation conditions), but prior to step (ii) (in which at least a portion of the product of step (i) is converted into the intermediate comprising R,R- and S,S-lactide). Alkyl alcohol and/or co-solvent may also be recovered and recycled. For example, these components may be separated from the products of the process by distillation and fed back into the process. Ketone solvents such as acetone have boiling points such that they can readily be separated from alkyl R-lactate and alkyl S,S-lactyllactate by distillation, allowing recycling of the solvent. For example, a product mixture comprising alkyl R-lactate (e.g. n-butyl R-lactate), alkyl S,S-lactyllactate (e.g. n-butyl S,S-lactyllactate), alkyl alcohol (e.g. n-butanol) and co-solvent (e.g.acetone) is separated by a two step distillation process in which alkyl alcohol and co-solvent are separated from alkyl R-lactate and alkyl S,S-lactyllactate in a first distillation step , and in which alkyl R-lactate is separated from alkyl S,S-lactyllactate in a second distillation step. In the case where the product mixture contains unreacted alkyl R,R-lactyllactate, then alkyl alcohol, co-solvent and alkyl R-lactate mat be separated from alkyl R,R-lactyllactate also by distillation, for example by a two step distillation process as discussed above.

Preferred embodiments of the invention include the following:
i) producing oligomeric S-lactic acid from S-lactic acid (for example S-lactic acid obtained by fermentation, or by hydrolysis of poly S-lactic acid, or by hydrolysis of alkyl S,S-lactyllactate, or by hydrolysis of alkyl S-lactate), subjecting oligomeric S-lactic acid to stereoisomerisation conditions to produce oligomeric lactic acid, converting at least a portion of the oligomeric lactic acid into R,R- and S,S-lactide together with R,S-lactide, separating R,R- and S,S-lactide from R,S-lactide (e.g. by distillation or recrystallisation), reacting the R,R- and S,S-lactide with an alkyl alcohol to produce a mixture containing alkyl S,S-lactyllactate and alkyl R,R-lactyllactate, reacting the mixture with alkyl alcohol in the presence of an enzyme to produce alkyl S,S-lactyllactate and alkyl R-lactate, separating alkyl R-lactate from alkyl S,S-lactyllactate (e.g. by distillation), and recovering alkyl R-lactate and/or alkyl S,S-lactyllactate. In that embodiment, alkyl S,S-lactyllactate and/or R,S-lactide produced by the process may optionally be recycled to the invention, (for example the alkyl S,S-lactyllactate may be hydrolysed to produce further S-lactic acid, which may then be returned to the process by combining it with the other S-lactic acid feedstock); or
ii) producing oligomeric S-lactic acid from alkyl S-lactate (for example methyl, ethyl, propyl, butyl or benzyl S-lactate), subjecting oligomeric S-lactic acid to stereoisomerisation conditions to produce oligomeric lactic acid, converting at least a portion of the oligomeric lactic acid into R,R- and S,S-lactide together with R,S-lactide, separating R,R- and S,S-lactide from R,S-lactide (e.g. by recrystallisation and/or distillation), reacting the R,R- and S,S-lactide with an alkyl alcohol to produce a mixture containing alkyl S,S-lactyllactate and alkyl R,R-lactyllactate, reacting the mixture with alkyl alcohol in the presence of an enzyme to produce alkyl S,S-lactyllactate and alkyl R-lactate, separating alkyl R-lactate from alkyl S,S-lactyllactate (e.g. by distillation), and recovering alkyl R-lactate and/or alkyl S,S-lactyllactate. In that embodiment, alkyl S,S-lactyllactate and/or R,S-lactide produced by the process may optionally be recycled to the invention, (for example the alkyl S,S-lactyllactate may be alcoholised to produce further alkyl S-lactate, which may then be returned to the process by combining it with the other alkyl S-lactate feedstock). In those preferred embodiments, where the product mixture contains unreacted alkyl R,R-lactyllactate,then alkyl alcohol, co-solvent and alkyl R-lactate may be separated from alkyl R,R-lactyllactate also, for example by distillation.

It will be understood that a process based on using an R-lactic acid-based feedstock, rather than an S-lactic acid-based feedstock, can also provide useful products, i.e. products containing the S-lactic acid building block (e.g. alkyl S,S-lactyllactate). Thus, in another aspect, the invention provides a process for producing alkyl S,S-lactyllactate comprising: producing an intermediate comprising R,R- and S,S-lactide from an initial compound comprising substructure (IP) 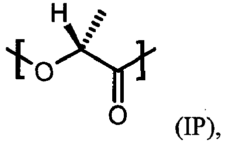 said process being as defined in claim 19. In that process, the initial compound may for example be poly R-lactic acid, oligomeric R-lactic acid, R-lactic acid or a salt thereof, alkyl R-lactate (e.g. methyl R-lactate), alkyl R,R-lactyllactate and/or R,R-lactide. Again, a single initial compound or a combination of initial compounds may be used. Other preferences mentioned above relating to processes based on an initial compound comprising substructure (I) (e.g. preferences concerning the stereoisomerisation catalyst, transesterification catalyst, alkyl alcohol, co-solvent, enzyme, and temperatures) are also applicable to processes based on an initial compound comprising substructure (IP).

Processes based on an initial compound comprising substructure (IP) again offer the advantage of allowing for recovery and recycling of reagents and catalysts used in the process, and also for recycling of lactic acid-derived byproducts produced by the process. In particular R,S-lactide and/or alkyl R-lactate may be recycled. R,S-lactide may be separated from R,R- and S,S-lactide and recycled, e.g. by distillation and/or by crystallisation. The R,S-lactide may then be recycled, for example by contacting the R,S-lactide with a tertiary amine stereoisomerisation catalyst so as to produce further R,R- and S,S-lactide (together with R,S-lactide), which can be combined with other intermediate streams, and allows the production of additional alkyl S,S-lactyllactate. Alkyl R-lactate may also be recovered and optionally recycled to the process. For example, alkyl R-lactate may be separated from alkyl S,S-lactyllactate by distillation. In the case where the initial compound is oligomeric R-lactic acid, which oligomeric R-lactic acid is produced from an R-lactic acid feedstock (e.g. by heating R-lactic acid under dehydrating conditions), the alkyl R-lactate may be hydrolysed to produce further R-lactic acid, which may then be returned to the process by combining it with the other R-lactic acid feedstock. Alternatively, the alkyl R-lactate may be converted to oligomeric R-lactic acid (e.g. by heating and distilling off alcohol) which may be returned to the process by combining it with other oligomeric R-lactic acid feedstocks.

Tertiary amine stereoisomerisation catalyst, alkyl alcohol and/or co-solvent may also be recycled as described above for processes based on an initial compound comprising substructure (I).

A preferred embodiment of the process of the invention is shown in Figure 1. An S-lactic acid stream **1** is introduced to oligomerisation reactor **10.** Heat is supplied to reactor **10** and water is removed by distillation as S-lactic acid is converted to oligomeric S-lactic acid, with concomitant removal of water. Stream **2** containing oligomeric S-lactic acid stream is then passed to stereoisomerisation reactor **20,** and is contacted with a tertiary amine (e.g. triethylamine) at elevated temperature to produce oligomeric lactic acid (i.e. the chiral centres in the oligomer are randomised). Stream **3** containing oligomeric lactic acid is separated from tertiary amine by distillation via distillation column **30.** The separated tertiary amine **10A** may be recycled by introduction into either stereoisomerisation reactor **20** or stereoisomerisation reactor **90** (discussed below). Stream **4** containing purified oligomeric lactic acid is then introduced into depolymerisation/lactide formation reactor 40 and is subjected to heating in the presence of a Lewis acid catalyst, such as tin (II) bis(2-ethylhexanoate), to produce a lactide mixture (R,R-, S,S- and R,S-lactide), which may for example be removed under vacuum at high temperature. Stream 5 containing the lactide mixture is introduced to recrystallisation reactor **50** and purified by crystallisation, (e.g. from ethyl acetate), resulting in separation of R,R- and S,S-lactide from R,S-lactide.

The process incorporates a *meso*-lactide recycling module, in which stream **11** containing the separated R,S-lactide byproduct is introduced to stereoisomerisation reactor **90** and contacted with tertiary amine at elevated temperature, resulting in a further lactide stream **5A** containing R,R-lactide, S,S-lactide, and R,S-lactide. Lactide stream **5A** is recycled to the process, (for example by introduction into recrystallisation reactor **50** and combination with lactide stream **5** or by hydrolysis and addition into oligomerisation reactor **10** (not shown).

Purified lactide stream **6** containing R,R- and S,S-lactide is introduced to alcoholysis/resolution reactor **60,** and the R,R- and S,S-lactide is reacted with alkyl alcohol (e.g. n-butanol) in the presence of enzyme (e.g. *Candida antartica* lipase B) and a co-solvent (e.g. acetone), to produce alkyl R-lactate and alkyl S,S-lactyllactate. Stream **7** containing the alkyl R-lactate and alkyl S,S-lactyllactate is then passed to distillation column **70** and the alkyl R-lactate and alkyl S,S-lactyllactate are separated by distillation. Alcohol **13** and co-solvent **14** are also separated from the lactate species, and may be recycled to reactor **60.** Optionally, the alcohol and co-solvent may be removed in a first distillation column, and alkyl R-lactate may be separated from alkyl S,S-lactyllactate using a second distillation column (not shown).

The process also incorporates an alkyl S,S-lactyllactate recycling module. Stream **12** containing alkyl S,S-lactyllactate is introduced to hydrolysis reactor **100** and converted to S-lactic acid and higher oligomers by hydrolysis. Stream **1A** containing the S-lactic acid is then recycled to the process via oligomerisation reactor **10** where it is combined with S-lactic acid stream **1.** The alcohol produced by hydrolysis of alkyl S,S-lactyllactate may also be recovered as alcohol stream **13A** and reintroduced into alcoholysis reactor **60.**

Stream **8** containing alkyl R-lactate is introduced to hydrolysis reactor **80,** and is converted to R-lactic acid **9** by hydrolysis. Again, alcohol produced by hydrolysis may be recovered as alcohol stream **13B** and reintroduced into alcoholysis reactor **60.**

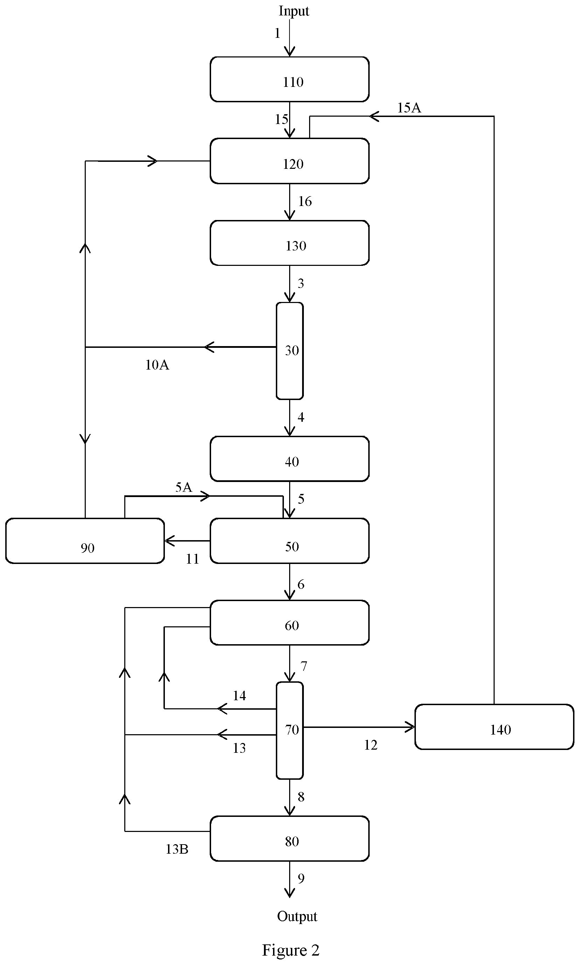

A variant of the process, based on racemisation of alkyl S-lactate, is shown in Figure 2. In that embodiment, stream **1** containing S-lactic acid is introduced to esterification reactor **110,** in which S-lactic acid is converted to alkyl S-lactate, for example by reaction with alcohol by heating in the presence of an acid catalyst. Stream **15** containing alkyl S-lactate is passed to stereoisomerisation reactor **120,** and the alkyl S-lactate is contacted with a tertiary amine (e.g. triethylamine) at elevated temperature to produce alkyl lactate. Stream **16** containing the alkyl lactate is then introduced to oligomerisation reactor **130.** Heat is supplied to reactor **130** and alcohol is removed by distillation, as the alkyl lactate is converted to oligomeric lactic acid. Oligomeric lactic acid stream **3** is then passed to distillation column **30,** and the remainder of the process is as described for the process shown in Figure 1, with the exception of the alkyl S,S-lactyllactate recycling module. Stream **12** containing alkyl S,S-lactyllactate is introduced to alcoholysis reactor **140** and converted to alkyl S-lactate (e.g. the alkyl S,S-lactyllactate is reacted with alkyl alcohol by heating in the presence of an acid catalyst). Alkyl S-lactate stream **15A** can then be combined with alkyl S-lactate stream 15 and recycled to the process.

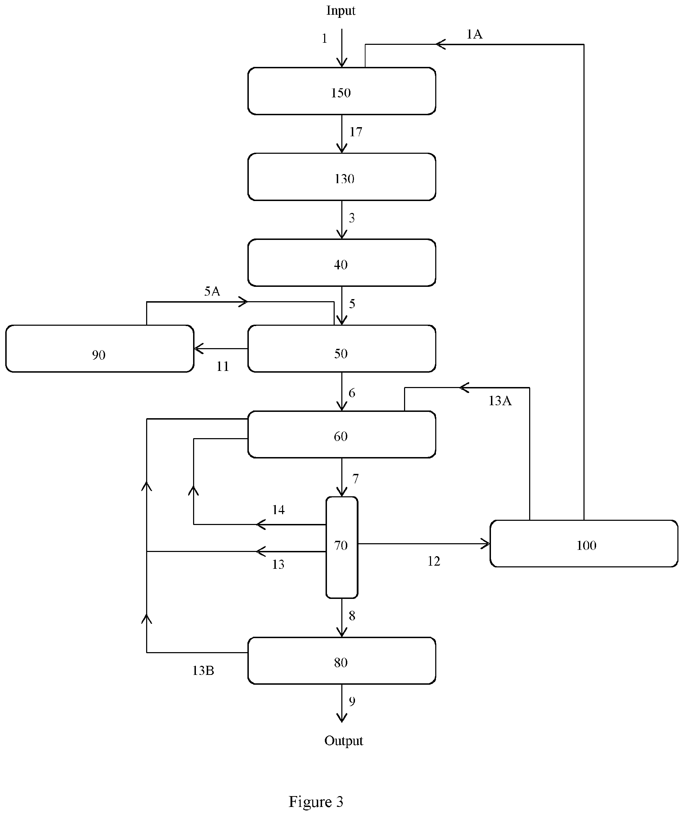

A further process variant is shown in Figure 3. Stream **1** containing S-lactic acid is introduced to esterification/racemisation reactor **150** and converted to racemic alkyl lactate, for example by reacting with alcohol by heating in the presence of an acid catalyst under conditions sufficient to racemise the chiral centre in S-lactic acid and/or alkyl S-lactate. Stream **17** containing the alkyl lactate is then introduced to oligomerisation reactor **130.** Heat is supplied to reactor **130** and alcohol is removed by distillation, as the alkyl lactate is converted to oligomeric lactic acid. Stream **3** containing oligomeric lactic acid is then introduced into depolymerisation/lactide formation reactor **40** and is subjected to heating in the presence of a Lewis acid catalyst, such as tin (II) bis(2-ethylhexanoate), to produce a lactide mixture (R,R-, S,S- and R,S-lactide). Stream 5 containing the lactide mixture is introduced to crystallisation reactor **50** and purified by crystallisation, (e.g. from ethyl acetate), resulting in separation of R,R- and S,S-lactide from R,S-lactide. The remainder of the process is as described in Figure 2, with the exception of the alkyl S,S-lactyllactate recycling module. Stream **12** containing alkyl S,S-lactyllactate is introduced to hydrolysis reactor **100** and is converted to S-lactic acid by hydrolysis. S-lactic acid stream **1A** is then combined with S-lactic acid stream **1** and recycled to the process.

It will be evident that certain stages of the process variants described above need not be carried out using separate reactors. For example, stereoisomerisation of oligomeric S-lactic acid and depolymerisation of the oligomer to produce a lactide mixture may be carried out in a single vessel, such as an evaporator unit. Oligomerisation of S-lactic acid to produce oligomeric S-lactic acid may also be carried out in the same vessel. It will also be understood that additional inputs not shown in the Figures, such as solvent, amine, enzyme, may be introduced at various stages. Similarly, the process of the invention may involve additional processing steps, for example additional neutralisation and/or separation and/or purification steps (such as filtration of the alkyl R-lactate/alkyl S,S-lactyllactate mixture to recover enzyme following the alcoholysis/resolution reaction).

The R-alkyl lactate obtained by the process of claim 1 can be converted into further useful downstream products by routine methods. Accordingly, the invention provides a process for producing R-lactic acid comprising producing R-alkyl lactate according to the process of claim 1, and converting the R-alkyl lactate into R-lactic acid, for example by hydrolysis of the alkyl ester group. In that case, the alkyl alcohol produced may also be recovered and recycled to the process.

The invention also provides a process for producing oligomeric R-lactic acid comprising producing R-alkyl lactate or producing R-lactic acid by the process of claim 1, and converting the R-alkyl lactate or R-lactic acid into oligomeric R-lactic acid. For example, the R-alkyl lactate or R-lactic acid may be converted into oligomeric R-lactic acid by heating, and removing the water or alcohol that is produced.

The invention also provides a process for producing R,R-lactide comprising producing R-alkyl lactate, or producing R-lactic acid, or producing oligomeric R-lactic acid by the process of claim 1, and converting the R-alkyl lactate, R-lactic acid or oligomeric R-lactic acid into R,R-lactide. For example, oligomeric R-lactic acid may be converted to R,R-lactide by heating in the presence of a transesterification catalyst (e.g. a Lewis acid catalyst comprising an oxide, alkoxide or carboxylate salt of a metal). An advantage of such processes involving subsequent conversion into R,R-lactide is that the production of alkyl R-lactate having lower enantiomeric excess can be tolerated. In such cases conversion into R,R-lactide will also result in the formation of R,S-lactide as a minor product (and very small quantities of S,S lactide). The R,S-lactide produced may readily be separated from the R,R-lactide, for example by distillation and/or crystallisation and/or washing.

Lactide may be polymerised to form polylactic acid. The invention therefore further provides a process for the production of poly-R-lactic acid, comprising producing R,R-lactide by a process according to claim 1, and converting the R,R-lactide into poly-R-lactic acid. This polymerisation may be carried out by contacting lactide with a suitable catalyst.

The invention also provides a process for producing stereocomplex lactic acid, comprising producing poly-R-lactic acid by the process of claim 1, and combining the poly-R-lactic acid with poly-S-lactic acid, for example using melt blending, to produce stereocomplex lactic acid.

The S,S-alkyl lactyllactate obtained by the process of claim 19 can also be converted into further useful downstream products by routine methods. Accordingly, disclosed is a process for producing S-lactic acid comprising producing S,S-alkyl lactyllactate by the process of claim 19, and converting the S,S-alkyl lactyllactate into S-lactic acid, for example by ester hydrolysis. In that case, the alkyl alcohol produced may also be recovered and recycled to the process.

Also disclosed is a process for producing oligomeric S-lactic acid comprising producing S,S-alkyl lactyllactate or producing S-lactic acid by the process of claim 19, and converting the S,S-alkyl lactyllactate or S-lactic acid into oligomeric S-lactic acid. For example, the S,S-alkyl lactyllactate or S-lactic acid may be converted into oligomeric S-lactic acid by heating, and removing the water or alcohol that is produced.

Also disclosed is a process for producing S,S-lactide comprising producing S-alkyl lactyllactate, or producing S-lactic acid, or producing oligomeric S-lactic acid by the process of claim 19, and converting the S,S-alkyl lactyllactate, S-lactic acid or oligomeric S-lactic acid into S,S-lactide. For example, oligomeric S-lactic acid may be converted to S,S-lactide by heating in the presence of a transesterification catalyst (e.g. a Lewis acid catalyst comprising an oxide, alkoxide or carboxylate salt of a metal).

Lactide may be polymerised to form polylactic acid, and disclosed is a process for the production of poly-S-lactic acid, comprising producing S,S-lactide by a process according to claim 19, and converting the S,S-lactide into poly-S-lactic acid. This polymerisation may be carried out by contacting lactide with a suitable catalyst.

Stereocomplex lactic acid may be prepared by producing poly-S-lactic acid by the process described above, and combining the poly-S-lactic acid with poly-R-lactic acid, for example using melt blending, to produce stereocomplex lactic acid.

Stereocomplex lactic acid may also be prepared by producing poly-S-lactic acid by a process described above, and poly-R-lactic acid by a process described above, and combining the poly-S-lactic acid with the poly-R-lactic acid, for example using melt blending, to produce stereocomplex lactic acid.

### Examples

### Example 1: S-Lactic acid racemisation using organic bases

S-Lactic acid solution (1.64kg, 80%wt in water, 14.6 mol) was heated to 120°C for 4 h at 100 mbar to remove the aqueous solvent (and some condensed water of oligomerisation). The temperature was then raised to 140°C for 60 minutes and then to 160°C for a further 60 minutes to form oligomeric S-lactic acid. The flask contents were then cooled to 140°C and placed under 1 atm of N₂. Triethylamine (147g, 0.1 molar equivalents) was added dropwise over 15 minutes and the reaction mixture was held at 140°C for 60 minutes to form oligomeric lactic acid. The reaction mixture was finally heated to 200°C and the pressure reduced to 100mbar to thoroughly remove the Et₃N.

Hydrolysis of the oligomeric product and analysis by chiral liquid chromatography confirmed the presence of equal quantities of S- and R- lactic acid components.

### Example 2: Conversion of S-lactic acid into S,S-, R,R-, and R,S-lactide using metal salts

S-lactic acid solution (85 wt % in water, 59mls/60.3g) and calcium stearate (3.45g, 0.01 molar equivalents) were stirred together in an unstoppered round-bottom flask fitted with internal thermocouple, and a magnetic stirrer bar. The mixture was heated to 160°C for 18hrs to remove water (lost to the atmosphere) forming oligomeric lactic acid.

The flask was then adapted for a distillation under reduced pressure by adding a still head, a condenser, a take-off arm connected to a vacuum pump and a collection flask (cooled in a solid CO₂ / acetone slush bath). A vacuum of 20mbar was applied and the flask temperature was slowly increased until lactide began to distil at 188°C. Over the course of the distillation the temperature was slowly increased up to a maximum of 242°C in order to keep the rate of lactide production approximately constant.

The yield of the collected lactide mixture was 30.65g (74.8%). Analysis by ¹H NMR and chiral gas chromatography confirmed a lactide composition of, 37.6% (S,S), 25.6% (R,R) and 36.8% meso (R,S).

### Example 3. Racemisation of Lactic Acid using Low pH Hydrothermolysis

S-Lactic acid solution was diluted in water to concentrations of 0.01, 0.1, 1.0, and 2.0M and was then pumped vertically upwards through a trace heated stainless steel reaction tube (0.5inch outer diameter, 36cm length, 35ml internal volume) at a flow rate of 1.2ml/min using an HPLC pump (calculated residency time in the reactor = 29 minutes). The tube was heated to temperatures of 250, 275, 300 and 325°C while the internal pressure was maintained at 150 bar via a back-pressure regulator attached to the top of the reaction tube after a cooling coil to quench reaction. The liquid vent from the regulator was collected and analysed by chiral liquid chromatography to determine the degree of racemisation.

| | Concentration of S-Lactic Acid (Molar) | | | | | | | |
|---|---|---|---|---|---|---|---|---|
| Temp (°C) | 0.01 | | 0.1 | | 1.0 | | 2.0 | |
| | % R-lactic acid | Total lactic acid yield% | % R-lactic acid | Total lactic acid yield% | % R-lactic acid | Total lactic acid yield% | % R-lactic acid | Total lactic acid yield% |
| 250 | 5.5 | 94 | 5.8 | 97 | 0.3 | 100 | 2.1 | 99 |
| 275 | 13.4 | 83 | 13.4 | 90 | 13.6 | 99 | 18.6 | 99 |
| 300 | 43.7 | 83 | 43.5 | 93 | 39.3 | 85 | 42.9 | 80 |
| 325 | 49.4 | 55 | 49.2 | 62 | 44.3 | 56 | 51.0 | 45 |

### Example 4. Racemisation of Lactic Acid using High pH Hydrothermolysis

S-Lactic acid solution was diluted in water to concentrations of 0.01, 0.1, 1.0, 2.0 and 4.0M and was then mixed with 1.1 equivalents of NaOH to form an alkaline solution of sodium lactate which was then pumped vertically upwards through a trace heated stainless steel reaction tube (0.5inch outer diameter, 36cm length, 35ml internal volume) at a flow rate of 1.2ml/min using an HPLC pump (calculated residency time in the reactor = 29 minutes). The tube was heated to temperatures of 250, 275, 300 and 325°C while the internal pressure was maintained at 150 bar via a back-pressure regulator attached to the top of the reaction tube after a cooling coil to quench reaction. The liquid vent from the regulator was collected and analysed by chiral liquid chromatography to determine the degree of racemisation.

A temperature of 325°C is required to achieve complete racemisation at all concentrations with or without NaOH. However, the addition of NaOH results in a much cleaner reaction, as seen by the lack of by-products in the LC trace, and consequently much higher yields of rac-lactate.

| | Concentration of S-Lactic Acid (Molar) | | | | | | | | | |
|---|---|---|---|---|---|---|---|---|---|---|
| Temp | 0.01 | | 0.1 | | 1.0 | | 2.0 | | 4.0 | |
| (°C) | % R-sodium lactate | Total sodium lactate yield% | % R-sodium lactate | Total sodium lactate yield% | % R-sodium lactate | Total sodium lactate yield% | % R-sodium lactate | Total sodium lactate yield% | % R-sodium lactate | Total sodium lactate yield% |
| 250 | 7.3 | 100 | 8.3 | 99 | 2.8 | 100 | 3.2 | 100 | 11.8 | 91 |
| 275 | 16.4 | 100 | 17.0 | 95 | 16.3 | 100 | 18.6 | 100 | 31.8 | 88 |
| 300 | 40.5 | 100 | 43.1 | 100 | 37.0 | 100 | 38.5 | 100 | 42.6 | 93 |
| 325 | 47.6 | 91 | 47.9 | 94 | 45.4 | 91 | 48.1 | 96 | 49.7 | 81 |

### Example 5: Racemization of S-Butyl Lactate with Potassium t-Butoxide at 20°C

Potassium t-butoxide (0.18g, 1.7mmol) was added to S-butyl lactate (5.0g, 34mmol, 99% e.e.) with stirring in a 10ml round bottom flask at room temperature. After stirring for 16h the colourless solution was cooled to room temperature, a drop of water was added to quench the KOtBu and a sample was taken for analysis by GC (Cyclosil B 30Mx0.32mmx 9.25um column) using acetone diluent and hexanol internal standard. The product was revealed to contain partially racemised S-butyl lactate (88% e.e.) and 12 % wt S,S-butyl lactyl lactate.

### Example 6: Racemization of S-Butyl Lactate with Potassium t-Butoxide at 50°C

Potassium t-butoxide (0.18g, 1.7mmol) was added to S-butyl lactate (5.0g, 34mmol, 99% e.e) with stirring in a 10ml round bottom flask at room temperature. The flask was then fitted with an air condenser and the mixture heated to 50°C for 16hrs. At the end of the reaction time the dark brown solution was cooled to room temperature, a drop of water was added to quench the KOtBu and a sample was taken for analysis by GC (Cyclosil B 30Mx0.32mmx 9.25um column) using acetone diluent and hexanol internal standard. The product was revealed to contain partially racemised S-butyl lactate (48% e.e.), 10 % wt S,S-butyl lactyl lactate, and a small quantity of R,R-, R,S- and S,R- butyl lactyl lactate.

### Example 7: Racemization of S-Butyl Lactate with Potassium t-Butoxide at 90°C

Potassium t-butoxide (0.18g, 1.7mmol) was added to S-butyl lactate (5.0g, 34mmol, 99% e.e) with stirring in a 10ml round bottom flask at room temperature. The flask was then fitted with an air condenser and the mixture heated to 90°C for 30mins. At the end of the reaction time the dark brown solution was cooled to room temperature, a drop of water was added to quench the KOtBu and a sample was taken for analysis by GC (Cyclosil B 30Mx0.32mmx 9.25um column) using acetone diluent and hexanol internal standard. The product was revealed to contain completely racemised butyl lactate (0% e.e.) with 10 % wt rac- and meso- butyl lactyl lactate.

### Example 8: Production of lactide

Oligomeric lactic acid (1068g) from Example 1 was heated to 120°C in a round bottom flask equipped with a still head connected by a trace-heated side-arm to a collection flask. Tin (II) bis(2-ethylhexanoate) (14.0g, 0.034 mol) was added. A vacuum of 15mbar was then applied and the vessel temperature was raised to 215°C for 90 mins causing a mixture of (S,S)-, (S,R)- and (R,R)- lactides to be distilled out of the flask. Total product yield was 830g (5.8 mol), ca. 80% of the theoretical yield and the composition of the distilled product was 35% (S,S)- : 35% (R,R)- : 30% (S,R)- lactide.

### Example 9: Purification of rac-lactide

To a mixture of (S,S)-, (S,R)- and (R,R)- lactides (725g 70 % rac- : 30 % meso-lactides) was added 620g of dry ethyl acetate (Aldrich). The mixture was heated to boiling at which point the lactide dissolved fully. The solution was then cooled using an ice bath, leading to precipitation of colourless crystals from the solution. The crystals were separated from the mixture by vacuum filtration, washed with 2 x 75ml of cold (-5°C) ethyl acetate and then dried. Product yield was 337 g (66 %) and purity was measured by chiral GC at 99% (S,S)- and (R,R)- lactides in equal proportions [<1% (S,R)-lactide].

### Examples 10-12: Stereoselective alcoholysis (batch)

A glass reactor was charged with racemic lactide (1 equivalent), n-butanol (1.5 equivalents), Novozym 435 (3% by weight of the racemic lactide) and varying amounts of a 90:10 (by weight) heptane/THF co-solvent (volumes based on the amount of n-butanol used). The mixture was stirred for 24 hours at room temperature and then analysed by chiral column chromatograpy for S-butyl lactate, R-butyl lactate, S,S-butyl lactyllactate and R,R-butyl lactyllactate so that the composition of the lactate components could be determined.

| **Example** | **Volume of heptane/THF** | **% R-butyl lactate** | **% S-butyl lactate** | **% S,S-butyl lactyllactate** | **% R,R-butyl lactyllactate** |
|---|---|---|---|---|---|
| 10 | 0 | 28 | 0 | 57 | 15 |
| 11 | 3.5 | 26 | 0 | 54 | 20 |
| 12 | 1.0 | 26 | 0 | 55 | 19 |

Note: percentages quoted are percentages of total peak area observed in chiral liquid chromatography chromatogram. If the reaction is allowed to progress in the presence of 3.0 equivalents of n-butanol, 49% alkyl R-lactate and 51% alkyl S,S-lactyllactate are observed. The experiments demonstrate that selective alcoholysis of alkyl R,R-lactyllactate to alkyl R-lactate takes place without significant alcoholysis of alkyl S,S-lactyllactate being observed.

### Examples 13-16: Stereoselective alcoholysis of rac-lactide in butanol (batch)

In a series of experiments 2.89 g of racemic lactide, 100 mg of Novozym-435 (3.5% by weight lactide) and varying quantities of n-butanol (1.5 to 10 molar equivalents based on the racemic lactide) were mixed together in a glass reactor and stirred at a temperature of 60 °C for a period of up to 24 hours. Samples were removed at varying times and analysed by chiral HPLC and NMR for the presence of R- and S-n-butyl lactate. Using this information % yields of R-n-butyl lactate based on the R,R-component of the racemic lactide starting material were calculated and reported in the following table. In all cases no S-n-butyl lactate was detected and no yields are therefore reported.

| **Example** | **Time (h)** | **Yield of R-n-butyl lactate** | | | | | |
|---|---|---|---|---|---|---|---|
| | | **1.5 eq** | **2 eq** | **2.5 eq** | **3 eq** | **6 eq** | **10 eq** |
| 13 | 2 | 43.5% | 67% | 57.5% | 45% | 38.5% | 39.5% |
| 14 | 5 | 57% | 77.5% | 81% | 82% | 46% | 44% |
| 15 | 7 | 55% | 76.5% | 90% | 96.5% (after 8 hours) | 92.5% | 86.5% |
| 16 | 24 | 59% | 77% | 89% | Not measured | 99.5% | 97% |

### Example 17

Analogous experiments to those described in Examples 10 to 16 were carried out using IMMCALB-T2-150, IMMCALBY-T2-150, IMMCALB-T1-350, or a cross-linked aggregate of lipase B from *Candida antarctica,* as the enzyme in place of Novozym-435. Those enzymes showed similar levels of stereoselectivity to Novozym-435.

### Example 18: Stereoselective alcoholysis (batch)

A glass vessel was charged with *rac*-lactide (2.30g), Novozym 435 (115mg, 5 wt % with respect to lactide), *n*-butanol (2.9ml, 2:1 molar ratio wrt lactide) then acetone (6.8ml). The mixture was shook by hand at RT to 45°C to ensure that the lactide dissolves. Then the vessel was placed in a heated shaker at 45°C, 750rpm (*t* = *0*)*.* The reaction was monitored over 24 hrs. Samples were analysed by chiral gas chromatography to determine the (*S*)-butyl lactate, (*R*)-butyl lactate, (*S,S*)-butyl lactyllactate, (*R,R*)-butyl lactyllactate, (*S,S*)-lactide and (*R,R*)-lactide composition. After 24hrs the reaction reached 89% conversion to (*R*)-butyl lactate at an optical purity >99% *e.e.*

### Example 19: Stereoselective alcoholysis of rac-lactide in butanol / acetone mixture with recycle of enzyme (batch)

Rac-lactide (1.45 g, 10 mmol) was alcoholised with n-BuOH (2.75 ml, 30 mmol, 3 eq.) and Novozym-435 (200 mg, 14 %) for 7 h at 35 °C in the presence of 2.75 ml of acetone. After 7 h the reaction was stopped and analysed for conversion to R-butyl lactate. The reaction liquors were then carefully separated from the immobilised enzyme by syringe and the enzyme was washed with solvent and reused in a subsequent run. The enzyme was reused for 8 repeat runs. Conversion to R-butyl lactate after the 1^{st} run was 92% (of the theoretical yield) and conversion after the 8^{th} run was 79%.

### Example 20: Stereoselective alcoholysis of rac-lactide in butanol/acetone mixture with recycle of enzyme (continuous)

At regular intervals a 50:50 mixture of (S,S)- and (R,R)- lactide was dissolved in acetone at a concentration of 30% wt lactide in a 1 litre water heated jacketed vessel at 45 °C equipped with a reflux condenser. n-Butanol was then added to the lactide solution so that the n-BuOH/lactide molar ratio was 2:1 at 45 °C; under these conditions the lactide remains in solution. Typical batches were prepared to supply the reaction rig with sufficient substrate to operate for at least 24 h.

The contents were then fed through a 400 mm length reflux column, the exterior collar of which was heated to 45 °C using recirculated heated water. The column was fitted directly onto a glass adaptor containing a 5 g packed bed of Novozym-435 (supported *Candida antarctica* Lipase B). The solution was fed through the column using a Watson Marlow 120S peristaltic pump and 1.6 mm ID Marprene tubing. Once passed through the enzyme bed the product mixture was collected and samples analysed by gas chromatography. Flow of reactants over the enzyme bed was adjusted to achieve a conversion of (R,R)-butyl lactyllactate into R-butyl lactate in the region 80 - 90%. Even after three months continuous operation conversions were >80% and the optical purity of the R-butyl lactate > 99% e.e.

### Example 21: Stereoselective alcoholysis of rac-lactide in butanol/methyl ethyl ketone (MEK) mixture with recycle of enzyme (continuous)

A solution of 10 g rac-lactide, 15 g BuOH, (3 Eq), and 50 g MEK (a ratio of 1:1.5:5) was passed through a steel column containing 0.500 g Novozym-435 immobilised *Candidia antarctica* Lipase B over a period of 60 h. Samples for analysis were taken at 2 hourly intervals from the feed and from the output of the column and the concentrations of (*S*)-butyl lactate, (*R*)-butyl lactate, (*S,S*)-butyl lactyllactate, (*R,R*)-butyl lactyllactate, (*S,S*)-lactide and (*R,R*)-lactide were determined by chiral liquid chromatography (no S-butyl lactate was detected). The conversion remained steady at 85% and the R-butyl lactate products were all >99% enantiomeric excess.

### Example 22: Distillation to separate butanol, butyl lactate and butyl lactyl lactate

150 g racemic *n*-butyl lactyllactate was gently stirred at 50 °C in the presence of 7.5 g Novozym-435 (5 % by weight of substrate) and 195 ml *n*-butanol for 6 hours, taking the conversion of (R,R)-n-butyl lactyllactate into (R)-n-butyl lactate to in excess of 95%. The immobilised enzyme was then removed by filtration and the bulk of the excess *n*-butanol removed by rotary evaporation at 50 °C/25 mbar. A portion of the residue (100 ml; 90g) was transferred to a round-bottomed flask equipped with a distillation column packed with Raschig rings, a still-head and condenser. Distillation was then carried out at 30 mbar, the vacuum being maintained by a Vacuubrand CVC 2000 controller.

Fractions were collected as summarised in the table below. Fractions 2 and 3 contained (R)-*n*-butyl lactate but no n-butyl lactylactate. Fraction 3 contained (R)-*n*-butyl lactate in > 98% ee. The results demonstrate that separation of alkyl lactate from alkyl lactylactate by distillation is possible without significant loss of enantiomeric purity.

| **Fraction** | **Still-head temperature range (°C)** | **Mass of fraction (g)** | **Composition of fraction** |
|---|---|---|---|
| Post-reaction | - | 90.0 | n-butanol (excess - not measured) |
| | | | (R)-n-butyl lactate (49%) |
| | | | (R,R)-n-butyl lactyllactate (1%) |
| | | | (S,S)-n-butyl lactyllactate (50%) |
| 1 | 50-52 | 29.0 | n-butanol (99%) |
| | | | (R)-n-butyl lactate (1%) |
| 2 | 52-100 | 7.4 | n-butanol (38%) |
| | | | (R)-n-butyl lactate (62%) |
| 3 | 100 | 22.1 | (R)-n-butyl lactate (>99%) |
| 4 | 100-154 | 14.7 | (R)-n-butyl lactate (34%) |
| | | | (R,R)-n-butyl lactyllactate (3%) |
| | | | (S,S)-n-butyl lactyllactate (63%) |
| 5 | 154-160 | 11.6 | n-butanol (0.2%) |
| | | | (R)-n-butyl lactate (0.2%) |
| | | | (R,R)-n-butyl lactyllactate (2%) |
| | | | (S,S)-n-butyl lactyllactate (97%) |
| Pot residue | - | 4.7 | (S,S)-n-butyl lactyllactate (»90%) |
| | | | Higher oligomers («10%) |

### Example 23: Distillation of acetone and butanol from butyl lactate and butyl lactyllactate

A 1 litre 3-necked glass flask was fitted with a magnetic stirrer bar and an insulated 20-plate Oldershaw column surmounted by a Perkin vacuum still head with 250 ml receiver. A feed point approximately half-way up the column allowed feedstock to be charged via a peristaltic pump using PharMed^{®} BPT peristaltic tubing. The flask was heated using an oil bath and vacuum was applied via a Teflon diaphragm pump, with a solid CO₂ cooled trap. The feedstock for this distillation consisted of acetone (49% wt); (R)-n-butyl lactate (21% wt); butanol (7% wt); (R,R)-n-butyl lactyllactate (3% wt) and (S,S)-n-butyl lactyllactate (19% wt). The remaining components included trace quantities of (S)-n-butyl lactate and both (S,S)- and (R,R)-lactides.

Initially, some extra butanol was added to the feed charge in order to establish continuous distillation conditions, since the amount of butanol present in the feedstock was low. Once this had been established (oil bath ∼135 °C, internal temp. ∼117 °C, still head temp. -77 °C, vacuum = 500 mBarA), the main feed was then charged at 2.5 - 5.0 ml/min. Fractions were collected as detailed below and analysed by chiral GC.

| | **Oil bath temp / °C** | **Internal temp / °C** | **Head temp. (°C)** | **Vacuum (mBarA)** | **Mass of fraction (g)** | **Composition by GC (%)** | | |
|---|---|---|---|---|---|---|---|---|
| **Fraction** | | | | | | **Acetone** | **Butanol** | **(R)-Butyl lactate** |
| **a)** | 135-149 | 117-135 | 70-76 | 500 | 45.3 | 99.5 | 0.0 | 0.5 |
| **b)** | 148-154 | 136-148 | 36-66 | 500 | 25.1 | 98.6 | 0.9 | 0.5 |
| **c)** | 147-153 | 138-147 | 30-36 | 500 | 10.1 | 96.1 | 3.4 | 0.5 |

From the 702 ml (609.5 g) of feedstock used, the composition of the resulting concentrated product (340.11 g) was: acetone (4.5%); (R)-n-butyl lactate (44.3%); n-butanol (4.7%); (R,R)-n-butyl lactyllactate (5.9%), (S,S)-n-butyl lactyllactate (39.0%) and (S)-n-butyl lactate (0.7%) with the remainder being (S,S)- and (R,R)-lactides.

The composition of the volatile products collected in the cold trap (59.7 g) was: acetone (89%) and butanol (10%) with the remaining 1% being both isomers of n-butyl lactate.

### Example 24: Distillation of acetone and butanol from butyl lactate and butyl lactyllactate

A continuous distillation set up was constructed comprising a 250 ml Hastalloy reboiler (with sightglass), a trace-heated 20-plate Oldershaw column surmounted by a Perkin vacuum still head with 250 ml receiver. There was a feed point approximately half-way up the column allowing feedstock to be charged via a peristaltic pump using PharMed^{®} BPT peristaltic tubing. The temperature of the reboiler and column heat tracing were electrically controlled. Vacuum was applied *via* a Teflon diaphragm pump, with a solid CO₂ cooled trap. The feedstock for this distillation (1050.0 g) consisted of: acetone (49% wt); (R)-n-butyl lactate (21% wt); butanol (7% wt); (R,R)-n-butyl lactyllactate (3% wt) and (S,S)-n-butyl lactyllactate (19% wt) with traces of (S)-n-butyl lactate and both (S,S)- and (R,R)-lactides. After the initial filling and conditioning of the column, the feedstock was fed in and rates and temperatures adjusted until steady continuous distillation was achieved. The optimum conditions were found to be vacuum = 100 mBarA; Reboiler temperature = 100 °C; Heat tracing = 65 °C; Feed rate = 4 ml/min.

These conditions were maintained throughout this distillation, and resulted in the product distribution detailed below. This procedure successfully concentrated the higher-boiling components [mainly (R)-n-butyl lactate and (S,S)-n-butyl lactyllactate] in the reboiler in high yields. Acetone and butanol recovery is also high and these solvents may be recycled to earlier stages of the overall process.

| **Details** | **Amount (g)** | **Composition by GC (%)** | | | | | | | |
|---|---|---|---|---|---|---|---|---|---|
| | | **Acetone** | **Butanol** | **(S)-BuLa** | **(R)-BuLa** | **(S,S)-BuLaLa** | **(R,R)-BuLaLa** | **(S,S)-Lactide** | **(R,R)-Lactide** |
| **Feedstock** | **1050.0** | **48.1** | **7.8** | **0.1** | **21.6** | **19.4** | **2.7** | **0.1** | **0.2** |
| | | | | | | | | | |
| **Distillates** | 63.4 | 11.0 | 53.6 | 0.2 | 28.9 | 5.6 | 0.8 | 0.0 | 0.0 |
| **Reboiler Fractions** | 335.3 | 0.4 | 3.5 | 0.2 | 45.0 | 44.3 | 6.3 | 0.1 | 0.3 |
| **Cold Trap** | 422.2 | 95.3 | 4.3 | 0.2 | 0.2 | 0.1 | 0.0 | 0.0 | 0.0 |
| **Sampling** | 126.8 | 4.6 | 10.3 | 0.7 | 24.9 | 24.4 | 3.5 | 0.0 | 0.2 |

| | | | | | | | | | |
|---|---|---|---|---|---|---|---|---|---|
| **BuLa = butyl lactate;** **BuLaLa = butyl lactyl lactate** | | | | | | | | | |

### Example 25: Distillation of butyl lactate from butyl lactyllactate

A continuous distillation apparatus was constructed comprising a 250 ml Hastalloy reboiler with sightglass fitted with a heated 20-plate Oldershaw column surmounted by a Perkin vacuum still head with 250 ml receiver. There was a feed point approximately half way up the column allowing feedstock to be charged via a peristaltic pump using PharMed^{®} BPT peristaltic tubing. The temperature of the reboiler and column heat tracing were electrically controlled. Vacuum was applied *via* a Teflon diaphragm pump, with a solid CO₂ cooled trap.

The feedstock for this distillation (740.5 g) consisted of: acetone (<0.5%); (R)-n-butyl lactate (46%); butanol (3%); (R,R)-n-butyl lactyllactate (6%) and (S,S)-n-butyl lactyllactate (44%) with trace quantities (<0.5%) of (S)-n-butyl lactate and both (S,S)- and (R,R)-lactides. After the initial filling and conditioning of the column, the feedstock was fed in and rates and temperatures adjusted until steady continuous distillation was achieved. The optimum conditions were found to be: Vacuum = 35 mBarA; Reboiler temperature = 150 °C; Heat tracing = 110 °C; Feed rate = 1 - 4 ml/min. These conditions were maintained throughout this distillation, and resulted in the product distribution detailed below:

| **Details** | **Mass (g)** | **Composition by GC (%)** | | | | | | | |
|---|---|---|---|---|---|---|---|---|---|
| | | **Acetone** | **BuOH** | **(S)-BuLa** | **(R)-BuLa** | **(S,S)-BuLaLa** | **(R,R)-BuLaLa** | **(S,S)-Lactide** | **(R,R)-Lactide** |
| **Feed** | **740.5** | **0.2** | **3.2** | **0.2** | **45.9** | **44.0** | **6.3** | **0.1** | **0.2** |
| | | | | | | | | | |
| **Distillate** | 277.6 | 0.0 | 5.0 | 0.4 | 93.9 | 0.5 | 0.1 | 0.0 | 0.1 |
| **Reboiler Fractions** | 389.6 | 0.0 | 0.3 | 0.2 | 17.7 | 70.7 | 10.3 | 0.3 | 0.5 |
| **Cold Trap** | 10.6 | 12.0 | 76.1 | 0.9 | 10.8 | 0.1 | 0.0 | 0.0 | 0.0 |
| **Sampling** | 53.1 | 2.6 | 0.7 | 0.2 | 16.1 | 69.5 | 10.3 | 0.2 | 0.4 |

The distilled product analysed as 93.9% (R)-butyl lactate; 0.4% (S)-butyl lactate, 5.0% butanol; 0.5% (S,S)-butyl lactyllactate; 0.1% (R,R)-butyl lactyllactate and 0.1% (R,R)-lactide.

### Example 26: Conversion of S-lactic acid to R-lactic acid

### Example 26a: Lactic Acid Racemisation

Commercially sourced S-lactic acid (1642g, 80% wt aqueous, 14.6 mol) was charged to a 2 litre flask fitted with mechanical stirrer and distillation take-off. The reaction mixture was heated to 120°C and held for 4 hours at 100 mbara to remove water. The mixture was then heated to 140°C for 60 minutes, then to 160°C for a further 60 minutes in order to build a low molecular weight oligomer and remove water of condensation formed during the reaction. After this period the oligomer was cooled to 140°C and the vacuum was released. Triethylamine (147g 1.45 mol) was added over 15 minutes and the reaction mix was held at this temperature for 60 minutes in order to racemise the oligomer to R- and S- lactate repeat units. The reaction mixture was then heated to 200°C at 100 mbara for 1 hour to remove the Et₃N to yield 1068 g of racemic oligomeric lactic acid.

### Example 26b: Rac / Meso- Lactide Preparation

Racemic oligomeric lactic acid (1068g) was heated to 120°C in a 2 litre flask fitted with mechanical stirrer and a heated take-off arm to an ice-cooled receiver. When the mixture was mobile and homogenous, tin(II) bis(2-ethylhexanoate) (14g, 0.034 mol) was added, a vacuum of 15mbar was applied and the vessel temperature was raised to 215°C. Under these conditions a mixture of (S,S)-, (S,R)- and (R,R)- lactides distilled from the mixture over approximately 90 minutes until the pot residue was depleted. Total product yield was 830g (5.8 mol), i.e. 79% of the theoretical yield from S-lactic acid used in example 26a.

### Example 26c: Rac-Lactide purification

To a mixture of (S,S)-, (S,R)- and (R,R)- lactides (725g, 5.0 mol, 70% rac : 30% meso lactide) was added 620g of dry ethyl acetate. The mixture was heated to boiling at which point the lactide dissolved fully. The mixture was then cooled slowly to -5°C using an ice/water bath during which time an amount of lactide precipitated from the solution. The solid produced was separated from the mixture by vacuum filtration. The product was washed on the filter with 2 aliquots (2 x 75ml) of ethyl acetate at -5°C before being dried. Product yield was 337g (66%) and purity was measured by chiral GC at 99% w/w (R,R) and (S,S)-lactides in equal proportions (<1% meso).

### Example 26d: Conversion of rac-lactide to R-butyl lactate

220 g of rac-lactide was dissolved in acetone at a concentration of 30% wt lactide and held in a 1 litre water heated jacketed vessel at 45°C equipped with a reflux condenser. n-Butanol was added to the lactide solution at 45°C so that the n-BuOH/lactide molar ratio was 2:1.

The solution was fed through a 400mm long reflux column, heated via a water bath at 45°C and pumped through the column base containing a 5g packed bed of supported Novozyme 435 enzyme, using a Watson Marlow 120S peristaltic pump and 1.6 mm ID marprene tubing. The column temperature was maintained at 45°C using flexible trace heating cable

The flow rate was approximately 0.55 ml/minute and the experiment was continued for 30 hours. The product mixture was collected at the column exit and sampled at regular intervals. Analysis was carried out using chiral GC. The conversion of (R,R)-lactide to (R)-BuLa averaged at 95% throughout the 30h period. No (S)-BuLa was detected.

### Example 26e: Purification of R-Butyl Lactate - step 1

The reaction mixture from Example 26d was separated by vacuum fractional distillation in 2 distillation steps. The first of these removed acetone and butanol from the mixture and the second separated R-butyl lactate from S,S-butyl lactyllactate.

Acetone and butanol were therefore separated from (R)-butyl lactate and (S,S)-butyl lactyllactate using a 20 theoretical plate linch Oldershaw column fitted with trace heating, a vacuum still head with reflux control and a re-boiler. The column was fed via peristaltic pump at the 10^{th} plate. The distillation was performed at 100 mbara pressure, using a reboiler temperature of 100°C, column trace heating of 65°C and a feed rate of 4ml/min.

### Example 26f: Purification of R-Butyl Lactate - step 2

R-butyl lactate and S,S-butyl lactyllactate were then separated using a 20 theoretical plate 1" Oldershaw column fitted with trace heating, a vacuum still head with reflux control and a re-boiler. The column was fed via peristaltic pump at the 10^{th} plate.. The distillation was performed at 35 mbara pressure, using a reboiler temperature of 150°C, column trace heating of 110°C and a feed rate of between 1 and 4 ml/min. The distilled fraction comprised 94% wt R-butyl lactate, 1% wt S,S-butyl lactylactate and 5% wt n-butanol. The yield of R-butyl lactate from Example 26d was calculated at 77%.

### Example 26g: Hydrolysis of R-butyl lactate to R-lactic acid

R-Butyl lactate (104g, 0.71mol), deionised water (500g) and acidic Amberlyst 15 resin (2.5g) were added to a 1 litre flask fitted with a reflux condenser and Dean and Stark take-off trap. The mixture was heated to reflux, and the organic distillate was removed via the Dean and Stark trap, returning the water to the flask. The reaction was deemed to be complete when no further organic material collected in the trap.
The product was analysed as 88.8 % wt water, 11.0 % wt R-lactic acid and 0.2 % wt n-butanol.

## Claims

1. A process for producing alkyl R-lactate comprising: producing an intermediate comprising R,R- and S,S-lactide from an initial compound comprising substructure (I) 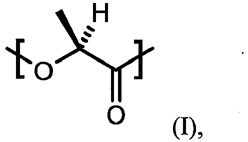 wherein the initial compound is subjected to stereoisomerisation conditions, and wherein the initial compound is contacted with a basic or acidic stereoisomerisation catalyst, resulting in a change from a first stereoisomeric distribution to a second different stereoisomeric distribution; and
reacting at least a portion of the intermediate with an alkyl alcohol to produce a product comprising alkyl R-lactate, wherein alkyl R-lactate is produced in the presence of an enzyme.

2. A process as claimed in claim 1, wherein the initial compound is contacted with a tertiary amine stereoisomerisation catalyst.

3. A process as claimed in claim 1 or claim 2, wherein the initial compound comprises oligomeric S-lactic acid, S-lactic acid or a salt thereof, alkyl S,S-lactyllactate and/or alkyl S-lactate, and wherein the intermediate is produced by:
(i) subjecting the initial compound to stereoisomerisation conditions; and
(ii) converting at least a portion of the product of step (i) into the intermediate.

4. A process as claimed in claim 3, wherein the initial compound comprises oligomeric-S-lactic acid, wherein the oligomeric S-lactic acid is produced from S-lactic acid, and wherein step (i) comprises contacting the oligomeric S-lactic acid with a tertiary amine stereoisomerisation catalyst at a temperature of from 100 to 200 °C to produce oligomeric lactic acid.

5. A process as claimed in claim 4, wherein step (ii) comprises converting at least a portion of the oligomeric lactic acid into R,R- and S,S-lactide by heating at a temperature of from 160 to 240°C in the presence of a Lewis acid catalyst comprising an oxide, alkoxide or carboxylate salt of a metal.

6. A process as claimed in any one of claims 2 to 5, wherein tertiary amine is recovered and recycled to the process.

7. A process as claimed in any preceding claim, wherein R,R- and S,S-lactide are separated from R,S-lactide, and R,S-lactide is recycled to the process.

8. A process as claimed in any preceding claim, wherein the alkyl alcohol is a C₂ to C₈ alkyl alcohol.

9. A process as claimed in any preceding claim, wherein the enzyme is a lipase.

10. A process as claimed in claim 9, wherein the enzyme is a *Candida antarctica* lipase B.

11. A process as claimed in any preceding claim, wherein alkyl R-lactate is separated from alkyl S,S-lactyllactate by distillation.

12. A process as claimed in claim 11, wherein alkyl S,S-lactyllactate is recovered and optionally recycled to the process.

13. A process as claimed in any preceding claim, wherein alkyl alcohol is recovered and recycled to the process.

14. A process for producing R-lactic acid comprising producing R-alkyl lactate according to any preceding claim, and converting the R-alkyl lactate into R-lactic acid.

15. A process for producing oligomeric R-lactic acid comprising producing R-alkyl lactate according to any one of claims 1 to 13, or producing R-lactic acid according to claim 14, and converting the R-alkyl lactate or R-lactic acid into oligomeric R-lactic acid.

16. A process for producing R,R-lactide comprising producing R-alkyl lactate according to any one of claims 1 to 13, or producing R-lactic acid according to claim 14, or producing oligomeric R-lactic acid according to claim 15, and converting the R-alkyl lactate, R-lactic acid or oligomeric R-lactic acid into R,R-lactide.

17. A process for producing poly-R-lactic acid comprising producing R,R-lactide according to claim 16, and converting the R,R-lactide into poly-R-lactic acid.

18. A process for producing stereocomplex lactic acid, comprising producing poly-R-lactic acid according to claim 17, and combining the poly-R-lactic acid with poly-S-lactic acid to produce stereocomplex lactic acid.

19. A process for producing alkyl S,S-lactyllactate comprising: producing an intermediate comprising R,R- and S,S-lactide from an initial compound comprising substructure (IP) 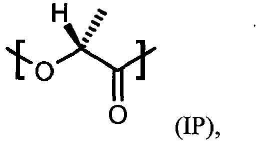 wherein the initial compound is subjected to stereoisomerisation conditions, and wherein the initial compound is contacted with a basic or acidic stereoisomerisation catalyst, resulting in a change from a first stereoisomeric distribution to a second different stereoisomeric distribution; and
reacting at least a portion of the intermediate with an alkyl alcohol to produce alkyl S,S-lactyllactate and alkyl R,R-lactyllactate, wherein the alkyl R,R-lactyllactate is reacted with alkyl alcohol in the presence of an enzyme to produce alkyl R-lactate.

20. A process as claimed in claim 19, wherein alkyl R-lactate is recovered and optionally recycled to the process.

## Patentansprüche

1. Ein Verfahren zur Herstellung von Alkyl-R-lactat, umfassend: Herstellen eines Zwischenprodukts, das R,R- und S,S-Lactid umfasst, aus einer Ausgangsverbindung, die die Unterstruktur (I) umfasst 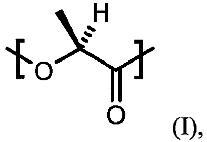 wobei die Ausgangsverbindung Stereoisomerisierungsbedingungen unterworfen wird, und wobei die Ausgangsverbindung mit einem basischen oder sauren Stereoisomerisierungskatalysator kontaktiert wird, was zu einem Wechsel von einer ersten stereoisomeren Verteilung zu einer zweiten, anderen stereoisomeren Verteilung führt, und
Umsetzen wenigstens eines Teils des Zwischenprodukts mit einem Alkylalkohol, um ein Produkt zu erzeugen, das Alkyl-R-lactat umfasst, wobei das Alkyl-R-lactat in Gegenwart eines Enzyms erzeugt wird.

2. Ein wie in Anspruch 1 beanspruchtes Verfahren, wobei die Ausgangsverbindung mit einem tertiär-Amin-Stereoisomerisierungskatalysator kontaktiert wird.

3. Ein wie in Anspruch 1 oder Anspruch 2 beanspruchtes Verfahren, wobei die Ausgangsverbindung oligomere S-Milchsäure, S-Milchsäure oder ein Salz davon, Alkyl-S-S-lactyllactat und/oder Alkyl-S-lactat umfasst, und wobei das Zwischenprodukt hergestellt wird durch:
(i) Unterwerfen der Ausgangsverbindung Stereoisomerisierungsbedingungen und
(ii) Umwandeln wenigstens eines Teils des Produkts von Schritt (i) in das Zwischenprodukt.

4. Ein wie in Anspruch 3 beanspruchtes Verfahren, wobei die Ausgangsverbindung oligomere S-Milchsäure umfasst, wobei die oligomere S-Milchsäure aus S-Milchsäure hergestellt ist, und wobei Schritt (i) das Kontaktieren der oligomeren S-Milchsäure mit einem tertiär-Amin-Stereoisomerisierungskatalysator bei einer Temperatur von 100 bis 200°C umfasst, um oligomere Milchsäure zu erzeugen.

5. Ein wie in Anspruch 4 beanspruchtes Verfahren, wobei Schritt (ii) das Umwandeln wenigstens eines Teils der oligomeren Milchsäure in R,R- und S,S-Lactid durch Erhitzen auf eine Temperatur von 160 bis 240°C in Gegenwart eines Lewis-Säure-Katalysators, umfassend ein Oxid, Alkoxid oder Carboxylatsalz eines Metalls, umfasst.

6. Ein wie in einem der Ansprüche 2 bis 5 beanspruchtes Verfahren, wobei das tertiäre Amin wiedergewonnen und in das Verfahren rückgeführt wird.

7. Ein wie in einem vorhergehenden Anspruch beanspruchtes Verfahren, wobei R,R- und S,S-Lactid von R,S-Lactid getrennt werden und das R,S-Lactid in das Verfahren rückgeführt wird.

8. Ein wie in einem vorhergehenden Anspruch beanspruchtes Verfahren, wobei der Alkylalkohol ein C₂- bis C₈-Alkylalkohol ist.

9. Ein wie in einem vorhergehenden Anspruch beanspruchtes Verfahren, wobei das Enzym eine Lipase ist.

10. Ein wie in Anspruch 9 beanspruchtes Verfahren, wobei das Enzym eine *Candida-antarctica*-Lipase B ist.

11. Ein wie in einem vorhergehenden Anspruch beanspruchtes Verfahren, wobei Alkyl-R-lactat von Alkyl-S,S-lactyllactat durch Destillation getrennt wird.

12. Ein wie in Anspruch 11 beanspruchtes Verfahren, wobei Alkyl-S,S-lactyllactat abgetrennt und gegebenenfalls in das Verfahren rückgeführt wird.

13. Ein wie in einem vorhergehenden Anspruch beanspruchtes Verfahren, wobei der Alkylalkohol wiedergewonnen und in das Verfahren rückgeführt wird.

14. Ein Verfahren zur Herstellung von R-Milchsäure, umfassend die Herstellung von R-Alkyllactat gemäß einem vorhergehenden Anspruch und die Umwandlung des R-Alkyllactats in R-Milchsäure.

15. Ein Verfahren zur Herstellung von oligomerer R-Milchsäure, umfassend die Herstellung von R-Alkyllactat gemäß einem der Ansprüche 1 bis 13 oder die Herstellung von R-Milchsäure gemäß Anspruch 14, und die Umwandlung des R-Alkyllactats oder der R-Milchsäure in oligomere R-Milchsäure.

16. Ein Verfahren zur Herstellung von R,R-Lactid, umfassend die Herstellung von R-Alkyllactat gemäß einem der Ansprüche 1 bis 13 oder die Herstellung von R-Milchsäure gemäß Anspruch 14 oder die Herstellung von oligomerer R-Milchsäure gemäß Anspruch 15, und die Umwandlung des R-Alkyllactats, der R-Milchsäure oder der oligomeren R-Milchsäure in R,R-Lactid.

17. Ein Verfahren zur Herstellung von Poly-R-milchsäure, umfassend die Herstellung von R,R-Lactid gemäß Anspruch 16 und die Umwandlung des R,R-Lactids, in Poly-R-milchsäure.

18. Ein Verfahren zur Herstellung stereokomplexer Milchsäure, umfassend die Herstellung von Poly-R-milchsäure gemäß Anspruch 17 und das Vereinen von Poly-R-milchsäure mit Poly-S-milchsäure, um stereokomplexe Milchsäure zu erzeugen.

19. Ein Verfahren zur Herstellung von Alkyl-S,S-lactyllactat, umfassend:
Herstellen eines Zwischenprodukts, das R,R- und S,S-Lactid umfasst, aus einer Ausgangsverbindung, die die Unterstruktur (IP) umfasst 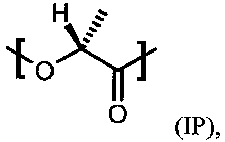 wobei die Ausgangsverbindung Stereoisomerisierungsbedingungen unterworfen wird, und wobei die Ausgangsverbindung mit einem basischen oder sauren Stereoisomerisierungskatalysator kontaktiert wird, was zu einem Wechsel von einer ersten stereoisomeren Verteilung zu einer zweiten, anderen stereoisomeren Verteilung führt, und
Umsetzen wenigstens eines Teils des Zwischenprodukts mit einem Alkylalkohol, um Alkyl-S,S-lactyllactat und Alkyl-R,R-lactyllactat zu erzeugen, wobei das Alkyl-R,R-lactyllactat mit Alkylalkohol in Gegenwart eines Enzyms umgesetzt wird, um Alkyl-R-lactat zu erzeugen.

20. Ein wie in Anspruch 19 beanspruchtes Verfahren, wobei Alkyl-R-lactat wiedergewonnen und gegebenenfalls in das Verfahren rückgeführt wird.

## Revendications

1. Procédé de production de R-lactate d'alkyle comprenant: la production d'un intermédiaire comprenant les R,R- et S,S-lactide à partir d'un composé initial comprenant la sous-structure (I) 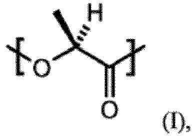 dans lequel le composé initial est soumis à des conditions de stéréoisomérisation et dans lequel le composé initial est mis en contact avec un catalyseur de stéréoisomérisation basique ou acide, ce qui entraîne un changement d'une première distribution stéréoisomère en une seconde distribution stéréoisomère différente ; et
la mise en réaction d'au moins une partie de l'intermédiaire avec un alcool alkylique pour produire un produit comprenant du R-lactate d'alkyle, le R-lactate d'alkyle étant produit en présence d'une enzyme.

2. Procédé selon la revendication 1, dans lequel le composé initial est mis en contact avec un catalyseur de stéréoisomérisation de type amine tertiaire.

3. Procédé selon la revendication 1 ou la revendication 2, dans lequel le composé initial comprend de l'acide S-lactique oligomère, de l'acide S-lactique ou un sel de celui-ci, du S,S-lactyllactate d'alkyle et/ou du S-lactate d'alkyle, et dans lequel l'intermédiaire est produit par :
(i) la soumission du composé initial à des conditions de stéréoisomérisation ; et
(ii) la conversion d'au moins une partie du produit de l'étape (i) en intermédiaire.

4. Procédé selon la revendication 3, dans lequel le composé initial comprend de l'acide S-lactique oligomère, l'acide S-lactique oligomère étant produit à partir d'acide S-lactique, et dans lequel l'étape (i) comprend la mise en contact de l'acide S-lactique oligomère avec un catalyseur de stéréoisomérisation de type amine tertiaire à une température de 100 à 200 °C pour produire l'acide lactique oligomère.

5. Procédé selon la revendication 4, dans lequel l'étape (ii) comprend la conversion d'au moins une partie de l'acide lactique oligomère en R,R- et S,S-lactide en chauffant à une température de 160 à 240 °C en présence d'un catalyseur de type acide de Lewis comprenant un sel d'un métal d'oxyde, d'alcoxyde ou de carboxylate.

6. Procédé selon l'une quelconque des revendications 2 à 5, dans lequel l'amine tertiaire est récupérée et recyclée vers le procédé.

7. Procédé selon l'une quelconque des revendications précédentes, dans lequel les R,R- et S,S-lactide sont séparés du R,S-lactide, et le R,S-lactide est recyclé vers le procédé.

8. Procédé selon l'une quelconque des revendications précédentes, dans lequel l'alcool alkylique est un alcool alkylique en C₂ à C₈.

9. Procédé selon l'une quelconque des revendications précédentes, dans lequel l'enzyme est une lipase.

10. Procédé selon la revendication 9, dans lequel l'enzyme est une lipase B *Candida antarctica.*

11. Procédé selon l'une quelconque des revendications précédentes, dans lequel le R-lactate d'alkyle est séparé du S,S-lactyllactate d'alkyle par distillation.

12. Procédé selon la revendication 11, dans lequel le S,S-lactyllactate d'alkyle est récupéré et de manière facultative recyclé vers le procédé.

13. Procédé selon l'une quelconque des revendications précédentes, dans lequel l'alcool alkylique est récupéré et recyclé vers le procédé.

14. Procédé de production d'acide R-lactique comprenant la production de R-lactate d'alkyle selon l'une quelconque des revendications précédentes, et la conversion du R-lactate d'alkyle en acide R-lactique.

15. Procédé de production d'acide R-lactique oligomère comprenant la production de R-lactate d'alkyle selon l'une quelconque des revendications 1 à 13, ou la production d'acide R-lactique selon la revendication 14, et la conversion du R-lactate d'alkyle ou de l'acide R-lactique en acide R-lactique oligomère.

16. Procédé de production de R,R-lactide comprenant la production de R-lactate d'alkyle selon l'une quelconque des revendications 1 à 13, ou la production d'acide R-lactique selon la revendication 14, ou la production d'acide R-lactique oligomère selon la revendication 15, et la conversion du R-lactate d'alkyle, de l'acide R-lactique ou de l'acide R-lactique oligomère en R,R-lactide.

17. Procédé de production de poly(acide R-lactique) comprenant la production de R,R-lactide selon la revendication 16, et la conversion du R,R-lactide en poly(acide R-lactique).

18. Procédé de production d'un stéréocomplexe d'acide lactique, comprenant la production de poly(acide R-lactique) selon la revendication 17, et la combinaison du poly(acide R-lactique) avec du poly(acide S-lactique) pour produire un stéréocomplexe d'acide lactique.

19. Procédé de production de S,S-lactyllactate d'alkyle comprenant : la production d'un intermédiaire comprenant les R,R- et S,S-lactide à partir d'un composé initial comprenant la sous-structure (IP) 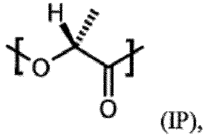 dans lequel le composé initial est soumis à des conditions de stéréoisomérisation et dans lequel le composé initial est mis en contact avec un catalyseur de stéréoisomérisation basique ou acide, ce qui entraîne un changement d'une première distribution stéréoisomère en une seconde distribution stéréoisomère différente ; et
la mise en réaction d'au moins une partie de l'intermédiaire avec un alcool alkylique pour produire du S,S-lactyllactate d'alkyle et du R,R-lactyllactate d'alkyle, le R,R-lactyllactate d'alkyle étant mis à réagir avec un alcool alkylique en présence d'une enzyme pour produire du R-lactate d'alkyle.

20. Procédé selon la revendication 19, dans lequel le R-lactate d'alkyle est récupéré et recyclé de manière facultative vers le procédé.
